# EUROPÄISCHE PATENTSCHRIFT

(11) **EP 3 057 967 B1**
(45) Veröffentlichungstag und Bekanntmachung des Hinweises auf die Patenterteilung: **11.10.2017**
(21) Anmeldenummer: 14783848.6
(22) Anmeldetag: 13.10.2014
(51) Int. Cl.: C07D 417/14, A01N 43/78

(54) **NEUE KRISTALLINE FORM VON 2-{3-[2-(1-{[3,5-BIS(DIFLUORMETHYL)-1H-PYRAZOL-1-YL]ACETYL}PIPERIDIN-4-YL)-1,3-THIAZOL-4-YL]-4,5-DIHYDRO-1,2-OXAZOL-5-YL}-3-CHLORPHENYLMETHANSULFONAT**
NEW CRYSTALLINE FORM OF 2-{3-[2-(1-{[3,5-BIS(DIFLUOROMETHYL)-1H-PYRAZOL-1-YL]ACETYL}PIPERIDIN-4-YL)-1,3-THIAZOL-4-YL]-4,5-DIHYDRO-1,2-OXAZOL-5-YL}-6-CHLOROPHENYLMETHANSULFONATE
NOUVELLE FORME CRYSTALLINE DE 2-{3-[2-(1-{[3,5-BIS(DIFLUOROMETHYL)-1H-PYRAZOL-1-YL]ACETYL}PIPERIDIN-4-YL)-1,3-THIAZOL-4-YL]-4,5-DIHYDRO-1,2-OXAZOL-5-YL}-6-CHLOROPHENYLMETHANSULFONATE

(30) Priorität: 17.10.2013 EP 13189076; 02.07.2014 EP 14175455
(43) Veröffentlichungstag der Anmeldung: 24.08.2016
(73) Patentinhaber: Bayer CropScience Aktiengesellschaft, 40789 Monheim am Rhein (DE)
(72) Erfinder: OLENIK, Britta, 46242 Bottrop (DE); HILLEBRAND, Stefan, 41462 Neuss (DE); WASNAIRE, Pierre, 40225 Düsseldorf (DE); WEISS, Martin, 53332 Bornheim/Sechtem (DE); WACHENDORFF-NEUMANN, Ulrike, 56566 Neuwied (DE)
(74) Vertreter: BIP Patents
(86) Internationale Anmeldenummer: PCT/EP2014/071874
(87) Internationale Veröffentlichungsnummer: WO 2015/055574

(56) Entgegenhaltungen:
- WO-A1-2012/025557
- CAIRA M R: "CRYSTALLINE POLYMORPHISM OF ORGANIC COMPOUNDS", TOPICS IN CURRENT CHEMISTRY, SPRINGER, BERLIN, DE, Bd. 198, 1. Januar 1998 (1998-01-01), Seiten 163-208, XP001156954, ISSN: 0340-1022, DOI: 10.1007/3-540-69178-2_5 ISBN: 978-3-540-36760-4

## Beschreibung

### Neue kristalline Form von 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat

Die vorliegende Erfindung betrifft eine neue kristalline Form von 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat (siehe Formel (I)), ein Verfahren zur Herstellung dieser neuen kristallinen Form, die vorteilhafte Verwendung zur Zubereitung von stabilen Anwendungsformen sowie deren Verwendung zur Bekämpfung von pflanzenpathogenen Schadpilzen in und/oder auf Pflanzen oder in und/oder auf Saatgut von Pflanzen.

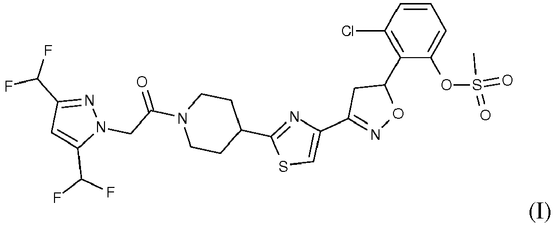

### 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat

Die Verbindung 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat sowie Methoden zur Herstellung dieser Verbindung sind bekannt. Auch ist bekannt, dass diese Verbindung fungizide Wirkung besitzt. So beschreibt beispielsweise die WO 2012/025557 erstmals die Herstellung dieser Verbindung sowie seine Verwendung zur Bekämpfung von pflanzenpathogenen Schadpilzen in und/oder auf Pflanzen oder in und/oder auf Saatgut von Pflanzen.

Außerdem ist bekannt, dass die Verbindung 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenylmethan-sulfonat in Mischungen mit anderen Fungiziden verwendet werden kann: WO2013/127704.

2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat aus WO 2012/025557 liegt als amorpher Feststoff vor. Er kann sowohl in Formulierungen, wie Emulsionskonzentraten (EC), in denen der Feststoff gelöst wird als auch in Formulierungen, in denen der Wirkstoff weiterhin in fester Form vorliegt (Feststoffformulierung) verwendet werden. Es handelt sich dabei beispielsweise um Granulate, verkapselte Granulate, Tabletten, wasserdispergierbare Granulate, wasserdispergierbare Tabletten, wasserdispergierbare Pulver oder wasserdispergierbare Pulver für Saatgutbehandlung, Staub-Formulierungen, Formulierungen, in denen der Wirkstoff in dispergierter Form vorliegt wie z.B.: Suspensionskonzentrate (SC), Öl basierte Suspensionskonzentrate, Suspoemulsionen, oder Suspensionskonzentrate für die Saatgutbehandlung.

Grundsätzlich sind Fesstoffformulierung von großer wirtschaftlicher Relevanz, da sie eine sehr gute Lagerstabilität aufweisen. Dieses ist aber nur bedingt der Fall, wenn der Wirkstoff in amorpher Form vorliegt und damit nicht in der thermodynamisch stabilsten Modifikation.

Da sich die ökologischen und ökonomischen Anforderungen an moderne Pflanzenschutzmittel laufend erhöhen, beispielsweise was Wirkspektrum, Aktivität und Aufwandmenge anbelangt, wäre es wünschenswert, neben der amorphen Form eine weitere Modifikation des Wirkstoffs 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat vorliegen zu haben, die in einer Feststoffformulierung zumindest in Teilbereichen Vorteile gegenüber der bekannten aufweist.

Überraschenderweise wurde nun eine neue kristalline Form von 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat gefunden, die die oben genannten Nachteile der Lagerstabilität in einer Feststoffformulierung vermeidet und verbesserte fungizide Aktivität gegenüber der amorphen Form in einer Fesstoffformulierung aufweist.

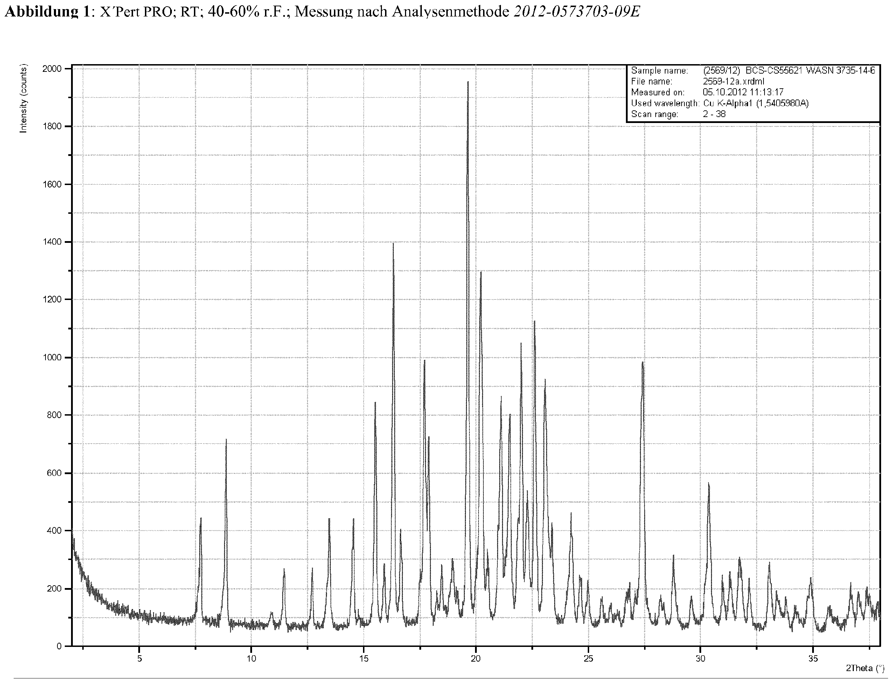

Die Erfindung bezieht sich deshalb auf die neue kristalline Form der Verbindung 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat, die dadurch gekennzeichnet ist, dass sie das Röntgen-Pulverdiffraktogramm (s. Abbildung 1) dieser Verbindung bei 25°C und Verwendung von Cu-Kα-Strahlung (1.54 Å) mindestens 3, insbesondere mindestens 4 oder 6 und vorzugsweise alle der folgenden 2Θ (2 Theta)-Werte aufweist (Tabelle 1):

**Tabelle 1:**

| |
|---|
| 2Θ = 7,75° ± 0,2 |
| 2Θ = 8,89 ± 0,2 |
| 2Θ = 10,91 ± 0,2 |
| 2Θ = 11,45 ± 0,2 |
| 2Θ = 12,72 ± 0,2 |
| 2Θ = 13,46 ± 0,2 |
| 2Θ = 14,54 ± 0,2 |
| 2Θ = 14,75 ± 0,2 |
| 2Θ = 15,53 ± 0,2 |
| 2Θ = 15,90 ± 0,2 |
| 2Θ = 16,33 ± 0,2 |
| 2Θ = 16,65 ± 0,2 |
| 2Θ = 17,49 ± 0,2 |
| 2Θ = 17,71 ± 0,2 |
| 2Θ = 17,89 ± 0,2 |
| 2Θ = 18,27 ± 0,2 |
| 2Θ = 18,47 ± 0,2 |
| 2Θ = 18,86 ± 0,2 |
| 2Θ = 18,99 ± 0,2 |
| 2Θ = 19,18 ± 0,2 |
| 2Θ = 19,63 ± 0,2 |
| 2Θ = 20,03 ± 0,2 |
| 2Θ = 20,22 ± 0,2 |
| 2Θ = 20,53 ± 0,2 |
| 2Θ = 21,11 ± 0,2 |
| 2Θ = 21,32 ± 0,2 |
| 2Θ = 21,50 ± 0,2 |
| 2Θ = 21,84 ± 0,2 |
| 2Θ = 22,01 ± 0,2 |
| 2Θ = 22,28 ± 0,2 |
| 2Θ = 22,61 ± 0,2 |
| 2Θ = 23,05 ± 0,2 |
| 2Θ = 23,39 ± 0,2 |
| 2Θ = 24,02 ± 0,2 |
| 2Θ = 24,22 ± 0,2 |
| 2Θ = 24,62 ± 0,2 |
| 2Θ = 24,97 ± 0,2 |
| 2Θ = 25,59 ± 0,2 |
| 2Θ = 25,99 ± 0,2 |
| 2Θ = 26,31 ± 0,2 |
| 2Θ = 26,67 ± 0,2 |
| 2Θ = 26,85 ± 0,2 |
| 2Θ = 27,07 ± 0,2 |
| 2Θ = 27,43 ± 0,2 |
| 2Θ = 28,21 ± 0,2 |
| 2Θ = 28,34 ± 0,2 |
| 2Θ = 28,78 ± 0,2 |
| 2Θ = 29,59 ± 0,2 |
| 2Θ = 30,19 ± 0,2 |
| 2Θ = 30,37 ± 0,2 |
| 2Θ = 30,96 ± 0,2 |
| 2Θ = 31,29 ± 0,2 |
| 2Θ = 31,70 ± 0,2 |
| 2Θ = 31,81 ± 0,2 |
| 2Θ = 32,16 ± 0,2 |
| 2Θ = 33,02 ± 0,2 |
| 2Θ = 33,39 ± 0,2 |
| 2Θ = 33,64 ± 0,2 |
| 2Θ = 33,79 ± 0,2 |
| 2Θ = 33,80 ± 0,2 |
| 2Θ = 34,20 ± 0,2 |
| 2Θ = 34,77 ± 0,2 |
| 2Θ = 34,89 ± 0,2 |
| 2Θ = 35,66 ± 0,2 |
| 2Θ = 35,95 ± 0,2 |
| 2Θ = 36,67 ± 0,2 |
| 2Θ = 37,03 ± 0,2 |
| 2Θ = 37,38 ± 0,2 |
| 2Θ = 37,51 ± 0,2 |
| 2Θ = 37,87 ± 0,2 |

Die neue kristalline Form weist ferner zu den 2 Theta-Werten die in Tabelle 2 aufgeführten Netzebenenabstände "d" auf.

**Tabelle 2: 2 Theta-Werte und Netzebenenabstände "d" der neuen kristallinen Form der Verbindung 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat, ermittelt durch Röntgen-Pulverdiffraktometrie.**

| **2Θ** | **d-Wert [Å]** |
|---|---|
| 7,75° ± 0,2 | 11,41 ± 0,1 |
| 8,89 ± 0,2 | 9,95 ± 0,1 |
| 10,91 ± 0,2 | 8,11 ± 0,1 |
| 11,45 ± 0,2 | 7,73 ± 0,1 |
| 12,72 ± 0,2 | 6,96 ± 0,1 |
| 13,46 ± 0,2 | 6,58 ± 0,1 |
| 14,54 ± 0,2 | 6,09 ± 0,1 |
| 14,75 ± 0,2 | 6,00 ± 0,1 |
| 15,53 ± 0,2 | 5,71 ± 0,1 |
| 15,90 ± 0,2 | 5,57 ± 0,1 |
| 16,33 ± 0,2 | 5,43 ± 0,1 |
| 16,65 ± 0,2 | 5,33 ± 0,1 |
| 17,49 ± 0,2 | 5,07 ± 0,1 |
| 17,71 ± 0,2 | 5,01 ± 0,1 |
| 17,89 ± 0,2 | 4,96 ± 0,1 |
| 18,27 ± 0,2 | 4,86 ± 0,1 |
| 18,47 ± 0,2 | 4,80 ± 0,1 |
| 18,86 ± 0,2 | 4,70 ± 0,1 |
| 18,99 ± 0,2 | 4,67 ± 0,1 |
| 19,18 ± 0,2 | 4,63 ± 0,1 |
| 19,62 ± 0,2 | 4,52 ± 0,1 |
| 20,03 ± 0,2 | 4,43 ± 0,1 |
| 20,22 ± 0,2 | 4,39 ± 0,1 |
| 20,53 ± 0,2 | 4,33 ± 0,1 |
| 21,11 ± 0,2 | 4,21 ± 0,1 |
| 21,32 ± 0,2 | 4,16 ± 0,1 |
| 21,50 ± 0,2 | 4,13 ± 0,1 |
| 21,84 ± 0,2 | 4,07 ± 0,1 |
| 22,01 ± 0,2 | 4,04 ± 0,1 |
| 22,28 ± 0,2 | 3,99 ± 0,1 |
| 22,61 ± 0,2 | 3,93 ± 0,1 |
| 23,05 ± 0,2 | 3,86 ± 0,1 |
| 23,39 ± 0,2 | 3,80 ± 0,1 |
| 24,02 ± 0,2 | 3,70 ± 0,1 |
| 24,22 ± 0,2 | 3,67 ± 0,1 |
| 24,62 ± 0,2 | 3,62 ± 0,1 |
| 24,97 ± 0,2 | 3,57 ± 0,1 |
| 25,59 ± 0,2 | 3,48 ± 0,1 |
| 25,99 ± 0,2 | 3,43 ± 0,1 |
| 26,31 ± 0,2 | 3,39 ± 0,1 |
| 26,67 ± 0,2 | 3,34 ± 0,1 |
| 26,85 ± 0,2 | 3,32 ± 0,1 |
| 27,07 ± 0,2 | 3,29 ± 0,1 |
| 27,43 ± 0,2 | 3,25 ± 0,1 |
| 28,21 ± 0,2 | 3,16 ± 0,1 |
| 28,34 ± 0,2 | 3,15 ± 0,1 |
| 28,78 ± 0,2 | 3,10 ± 0,1 |
| 29,59 ± 0,2 | 3,019 ± 0,1 |
| 30,19 ± 0,2 | 2,96 ± 0,1 |
| 30,37 ± 0,2 | 2,94 ± 0,1 |
| 30,96 ± 0,2 | 2,89 ± 0,1 |
| 31,29 ± 0,2 | 2,86 ± 0,1 |
| 31,70 ± 0,2 | 2,82 ± 0,1 |
| 31,81 ± 0,2 | 2,81 ± 0,1 |
| 32,16 ± 0,2 | 2,78 ± 0,1 |
| 33,02 ± 0,2 | 2,71 ± 0,1 |
| 33,39 ± 0,2 | 2,68 ± 0,1 |
| 33,64 ± 0,2 | 2,66 ± 0,1 |
| 33,79 ± 0,2 | 2,66 ± 0,1 |
| 33,80 ± 0,2 | 2,65 ± 0,1 |
| 34,20 ± 0,2 | 2,62 ± 0,1 |
| 34,77 ± 0,2 | 2,58 ± 0,1 |
| 34,89 ± 0,2 | 2,57 ± 0,1 |
| 35,66 ± 0,2 | 2,52 ± 0,1 |
| 35,95 ± 0,2 | 2,50 ± 0,1 |
| 36,67 ± 0,2 | 2,45 ± 0,1 |
| 37,03 ± 0,2 | 2,43 ± 0,1 |
| 37,38 ± 0,2 | 2,40 ± 0,1 |
| 37,51 ± 0,2 | 2,40 ± 0,1 |
| 37,87 ± 0,2 | 2,37 ± 0,1 |

Es wurde überraschend gefunden, dass 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat in der neuen kristallinen Form für die wirtschaftlich relevante Anwendung ausreichend thermodynamisch stabil ist und auch bei längerer Lagerung sich nicht in eine andere Form umwandelt.

Die Herstellung von Suspensionskonzentraten, Öl basierten Suspensionskonzentraten und z.B. wasserdispergierbaren Granulaten sowie ähnlicher Formulierungen zur Behandlung von Saatgut wird dadurch möglich.

Die Verbindung 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat in der neuen kristallinen Form eignet sich aufgrund ihrer Stabilität hervorragend für die Zubereitung von Mitteln zur Bekämpfung von pflanzenpathogenen Schadpilzen in und/oder auf Pflanzen oder in und/oder auf Saatgut von Pflanzen. Gegenstand der Erfindung sind daher auch Mittel zur Bekämpfung von pflanzenpathogenen Schadpilzen in und/oder auf Pflanzen oder in und/oder auf Saatgut von Pflanzen, welche die neue kristalline Form alleine oder in Mischung mit Hilfs- und Trägerstoffen, sowie in Mischung mit anderen Wirkstoffen enthalten.

Die Erfindung schließt auch Zusammensetzungen ein, die die Verbindung 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat in der neuen kristallinen Form enthalten. Bevorzugt sind Zusammensetzungen, die weniger als 20 Gew.-% der neuen feste Form, besonders bevorzugt weniger als 15 Gew.-%, ganz besonders bevorzugt weniger als 10 Gew.-%, insbesondere bevorzugt weniger als 5 Gew.-%, am meisten bevorzugt weniger als 4, 3, 2 oder 1 Gew.-% der neuen kristallinen Form enthalten. Die Bezeichnung "Zusammensetzung" umfasst auch Formulierungen und Anwendungsformen.

Gegenstand der Erfindung sind ferner auch Verfahren zur Herstellung von Mitteln zur Bekämpfung von pflanzenpathogenen Schadpilzen in und/oder auf Pflanzen oder in und/oder auf Saatgut von Pflanzen, welche die Verbindung 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat in der neuen kristallinen Form verwenden. Durch den Einsatz der Verbindung 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat in der neuen kristallinen Form wird die Sicherheit für Zubereitungen, die die Verbindung 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat enthalten, erhöht und somit das Risiko falscher Dosierungen verringert.

Die Verbindung 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat in der neuen kristallinen Form kann in bekannter Weise in die üblichen Formulierungen, wie Suspensionskonzentrate, Öl basierte Suspensionskonzentrate, kolloidale Konzentrate, dispergierbare Konzentrate, emulgierbare Konzentrate (Emulsionskonzentrate), Emulsionsbeizen, Suspensionsbeizen, Granulate, Mikrogranulate, Suspoemulsionen, wasserlösliche Granulate, wasserlösliche Konzentrate und wasserdispergierbare Granulate, wie auch entsprechender anwendungsfertiger Formulierungen (sogenannter ready-to-use Formulierungen) unter Verwendung geeigneter Hilfs- und Trägerstoffe oder Lösemittel überführt werden. Hierbei soll die wirksame Verbindung in einer Konzentration von etwa 0,0001 bis 90 Gew.-% der Gesamtmischung vorhanden sein, d.h. in Mengen, die ausreichend sind, um den notwendigen Dosierungsspiegel zu erreichen. Die Formulierungen werden beispielsweise hergestellt durch Verstrecken der Verbindung 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat in der neuen kristallinen Form mit Wasser, Lösemitteln und/oder Trägerstoffen, gegebenenfalls unter Verwendung von Emulgier- und/oder Dispergiermitteln, und/oder anderen Hilfsstoffen, wie z.B. Penetrationshilfsmitteln.

Bei der Herstellung von Suspensionskonzentraten, auch von solchen, die zur Saatgutbehandlung verwendet werden, werden im Allgemeinen neben dem Wirkstoff und einem Streckmittel (Wasser, Lösungsmittel oder Öl) weitere Hilfsmittel zugegeben. Zur Befeuchtung des Wirkstoffs in der kontinuierlichen Phase wird ein Netzmittel eingesetzt, zur Stabilisierung der Suspension in der flüssigen Phase werden Dispergierhilfsmittel verwendet, zum Emulgieren der nicht wässerige Phase werden bei Lösungsmittel oder Öl enthaltenden Suspensionskonzentraten Emulgiermittel eingesetzt. Sofern notwendig werden Frostschutzmittel, Biozide, Verdickungsmittel, Farbstoffe, Spreizmittel oder / und Aufnahmeförderer eingearbeitet.

Die neue feste Verbindung 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat wird durch das nachfolgend beschriebene Verfahren erhalten. Die Verbindung 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat wird wie beispielsweise in WO 2012/025557 beschrieben hergestellt. Das so erhaltene 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-6-chlorophenylmethan-sulfonat liegt im amorphen Zustand vor.

Zur Herstellung der erfindungsgemäßen Verbindung wird amorphes 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat in einem Lösungsmittel oder einem Lösungsmittelgemisch bei Temperaturen von mindestens 50°C, vorzugsweise von mindestens 80°C, suspendiert und langsam abgekühlt bis die gewünschten Kristalle ausfallen. Die Menge des verwendeten Lösungsmittels wird so gewählt, dass die Mischung während der Kristallisation gut rührbar bleibt.

Das langsame Abkühlen findet linear oder stufenweise statt. Bevorzugt findet das Abkühlen linear statt, wobei die Kristallisationslösung bei einer bestimmten Temperatur, vorzugsweise bei etwa 20°C für einige Stunden, vorzugsweise 5 bis 25 Stunden, besonders bevorzugt 15 bis 20 Stunden ganz besonders bevorzugt 16 bis 17 Stunden gerührt wird, bevor sie weiter abgekühlt werden bis dass die gewünschten Kristalle ausfallen.

Gegebenenfalls werden der Suspension Impfkristalle zugesetzt, um die Kristallisation zu beschleunigen. Das Zusetzen der Impfkristalle kann grundsätzlich während des Rührens geschehen.

Geeignete Lösungsmittel sind Halogenkohlenwasserstoffe (z.B. Chlorbenzol, Brombenzol, Dichlorbenzol, Chlortoluol, Trichlorbenzol), Ether (z.B. Ethylpropylether, Methyl-tert-butylether, n-Butylether, Anisol, Phenetol, Cyclohexylmethylether, Dimethylether, Diethylether, Dimethylglycol Diphenylether, Diproplether, Diisopropylether, Di-n-butylether, Diisobutylether, Diisoamylether, Ethylenglycoldimethylether, Isopropylethylether, Methyl-tert-butylether, Tetrahydrofuran, Methyl-Tetrahydrofuran, Dioxan, Dichlordiethylether, Methyl-THF und Polyether des Ethylenoxids und/oder Propylenoxids), Nitrokohlenwasserstoffe (z.B. Nitromethan, Nitroethan, Nitropropan, Nitrobenzol, Chlornitrobenzol, o-Nitrotoluol), aliphatische, cycloaliphatische oder aromatische Kohlenwasserstoffe (z.B. Pentan, n-Hexan, n-Heptan, n-Oktan, Nonan beispielsweise sogenannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Petrolether, Ligroin, Octan, Benzol, Toluol, Xylol), Ester (z.B. Malonester, Essigsäure-n-butylester (n-Butylacetat), Methylacetat, Ethylacetat, Isobutylacetat, Dimethylcarbonat, Diethylcarbonat, Dibutylcarbonat, Ethylencarbonat); und aliphatische Alkohole (z.B. Methanol, Ethanol, n-Propanol und Isopropanol und n-Butanol, tert.-Amylalkohol). Bevorzugte Lösungsmittel sind Ether, aromatische Kohlenwasserstoffe wie Benzol, Toluol, Xylol, Chlorbenzol, Ester und aliphatische Alkohole und Gemische davon. Besonders bevorzugte Lösungsmittel bzw. Lösungsmittelgemische sind Isopropanol, Toluol, Methyl-THF Diethylcarbonat, Chlorbenzol, n-Butylacetat und i-Butylacetat, n-Butanol, Ethanol, Malonsäureethylester, Methyl-t.butylether, sowie Mischungen aus Toluol und Butanol, Toluol und n-Butylacetat, Malonsäureethylester und Methyl-t.-butylether, und Butylacetat und Methyl-t.-butylether. Lösungsmittelmischungen mit mehr als 2 Komponenten sind auch möglich.

Neben den vorgenannten Formulierungen kann die Verbindung 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat in der neuen kristallinen Form in weitere Formulierungen überführt werden. Solche Formulierungen sind beispielsweise Lösungen, Emulsionen, Spritzpulver, wasser- und ölbasierte Suspensionen, Pulver, Stäubemittel, Pasten, lösliche Pulver, lösliche Granulate, Streugranulate, Suspensions-Emulsions-Konzentrate, Wirkstoff-imprägnierte Naturstoffe, Wirkstoff-imprägnierte synthetische Stoffe, Düngemittel sowie Feinstverkapselungen in polymeren Stoffen. Erfindungsgemäß bevorzugt sind Feststoffformulierungen.

Die Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Netzmitteln, Emulgiermitteln, Dispergiermitteln und/oder schaumzerstörenden Mitteln. Die Herstellung der Formulierungen erfolgt entweder in geeigneten Anlagen oder auch vor oder während der Anwendung.

Als Hilfsstoffe können solche Stoffe Verwendung finden, die geeignet sind, dem Mittel selbst oder und/oder davon abgeleitete Zubereitungen (z.B. Spritzbrühen, Saatgutbeizen) besondere Eigenschaften zu verleihen, wie bestimmte technische Eigenschaften und/oder auch besondere biologische Eigenschaften. Als typische Hilfsmittel kommen in Frage: Streckmittel, Lösemittel und Trägerstoffe.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Cyclohexanon), Ester (auch Fette und Öle) und (poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösungsmittel verwendet werden. Als flüssige Lösemittel kommen im wesentlichen in Frage: Aromaten, wie Xylol, Toluol, oder Alkylnaphthaline, aliphatische Kohlenwasserstoffe oder Paraffine, z.B. Erdölfraktionen, mineralische und pflanzliche Öle, Alkohole sowie deren Ether und Ester, Ketone wie Methylethylketon, Methylisobutylketon oder Cyclohexanon, stark polare Lösungsmittel, wie N-Methylpyrrolidon, Dimethylsulfoxid, sowie Wasser.

Erfindungsgemäß bedeutet Trägerstoff eine natürliche oder synthetische, organische oder anorganische Substanz, welcher fest oder flüssig sein kann, mit welchen die Wirkstoffe zur besseren Anwendbarkeit, insbesondere zum Aufbringen auf Pflanzen oder Pflanzenteile oder Saatgut, gemischt oder verbunden sind. Der feste oder flüssige Trägerstoff ist im Allgemeinen inert und sollte in der Landwirtschaft verwendbar sein.

Als feste oder flüssige Trägerstoffe kommen in Frage:
Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Sand, Bims, Sepiolith, Dolomit sowie synthetische Granulate aus anorganischen und organischen Mehlen sowie Granulate aus organischem Material wie Papier, Sägemehl, Kokosnussschalen, Maiskolben und Tabakstängeln; als Emulgier- und/oder schaumerzeugende Mittel kommen in Frage: Z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage nicht-ionische und/oder ionische Stoffe, z.B. aus den Klassen der Fettalkohol-POE- und/oder POP-Ether, Säure- und/oder POP- POE-Ester, Alkyl-Aryl- und/oder POP- POE-Ether, Fett- und/oder POP- POE-Addukte, POE- und/oder POP-Polyol Derivate, POE- und/oder POP-Sorbitan- oder Zucker-Addukte, Alky- oder Aryl-Sulfate, Sulfonate und Phosphate oder die entsprechenden PO-Ether-Addukte. Ferner können Einsatz finden Lignin und seine Sulfonsäure-Derivate, einfache und modifizierte Cellulosen, aromatische und/oder aliphatische Sulfonsäuren sowie deren Addukte mit Formaldehyd.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Weitere Additive können Duftstoffe, mineralische oder vegetabile gegebenenfalls modifizierte Öle, Wachse und Nährstoffe (auch Spurennährstoffe), wie Salze von Eisen, Mangan, Bor, Kupfer, Kobalt, Molybdän und Zink sein.

Weiterhin enthalten sein können Stabilisatoren wie Kältestabilisatoren, Konservierungsmittel, Oxidationsschutzmittel, Lichtschutzmittel oder andere die chemische und/oder physikalische Stabilität verbessernde Mittel.

Der Wirkstoffgehalt der aus den Formulierungen bereiteten Anwendungsformen kann in weiten Bereichen variieren. Die Wirkstoffkonzentration der Anwendungsformen liegt im Bereich von 0,00000001 bis 97 Gew.-% Wirkstoff, vorzugsweise im Bereich von 0,0000001 bis 97 Gew.-%, besonders bevorzugt im Bereich von 0,000001 bis 83 Gew.-% oder 0,000001 bis 5 Gew.-% und ganz besonders bevorzugt im Bereich von 0,0001 bis 1 Gew.-%.

Die erfindungsgemäße Verbindung 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat in der neuen kristallinen Form eignet sich bei guter Pflanzenverträglichkeit, günstiger Warmblütertoxizität und guter Umweltverträglichkeit zum Schutz von Pflanzen und Pflanzenorganen, zur Steigerung der Ernteerträge, Verbesserung der Qualität des Erntegutes und zur Bekämpfung von von pflanzenpathogenen Schadpilzen in und/oder auf Pflanzen oder in und/oder auf Saatgut von Pflanzen. Beispielhaft, aber nicht begrenzend, seien einige Erreger von pilzlichen Erkrankungen, die erfindungsgemäß behandelt werden können, genannt:
Erkrankungen, hervorgerufen durch Erreger des Echten Mehltaus wie z.B. Blumeria-Arten, wie beispielsweise Blumeria graminis; Podosphaera-Arten, wie beispielsweise Podosphaera leucotricha; Sphaerotheca-Arten, wie beispielsweise Sphaerotheca fuliginea; Uncinula-Arten, wie beispielsweise Uncinula necator;
Erkrankungen, hervorgerufen durch Erreger von Rostkrankheiten wie z.B. Gymnosporangium-Arten, wie beispielsweise Gymnosporangium sabinae; Hemileia-Arten, wie beispielsweise Hemileia vastatrix; Phakopsora-Arten, wie beispielsweise Phakopsora pachyrhizi oder Phakopsora meibomiae; Puccinia-Arten, wie beispielsweise Puccinia recondita, Puccinia graminis oder Puccinia striiformis; Uromyces-Arten, wie beispielsweise Uromyces appendiculatus;
Erkrankungen, hervorgerufen durch Erreger der Gruppe der Oomyceten wie z.B. Albugo-Arten, wie beispielsweise Albugo candida; Bremia-Arten, wie beispielsweise Bremia lactucae; Peronospora-Arten, wie beispielsweise Peronospora pisi oder P. brassicae; Phytophthora-Arten, wie beispielsweise Phytophthora infestans; Plasmopara-Arten, wie beispielsweise Plasmopara viticola; Pseudoperonospora-Arten, wie beispielsweise Pseudoperonospora humuli oder Pseudoperonospora cubensis; Pythium-Arten, wie beispielsweise Pythium ultimum;
Blattfleckenkrankheiten und Blattwelken, hervorgerufen durch z.B. Alternaria-Arten, wie beispielsweise Alternaria solani; Cercospora-Arten, wie beispielsweise Cercospora beticola; Cladosporium-Arten, wie beispielsweise Cladosporium cucumerinum; Cochliobolus-Arten, wie beispielsweise Cochliobolus sativus (Konidienform: Drechslera, Syn: Helminthosporium) oder Cochliobolus miyabeanus; Colletotrichum-Arten, wie beispielsweise Colletotrichum lindemuthanium; Cycloconium-Arten, wie beispielsweise Cycloconium oleaginum; Diaporthe-Arten, wie beispielsweise Diaporthe citri; Elsinoe-Arten, wie beispielsweise Elsinoe fawcettii; Gloeosporium-Arten, wie beispielsweise Gloeosporium laeticolor; Glomerella-Arten, wie beispielsweise Glomerella cingulata; Guignardia-Arten, wie beispielsweise Guignardia bidwelli; Leptosphaeria-Arten, wie beispielsweise Leptosphaeria maculans; Magnaporthe-Arten, wie beispielsweise Magnaporthe grisea; Microdochium-Arten, wie beispielsweise Microdochium nivale; Mycosphaerella-Arten, wie beispielsweise Mycosphaerella graminicola, Mycosphaerella arachidicola oder Mycosphaerella fijiensis; Phaeosphaeria-Arten, wie beispielsweise Phaeosphaeria nodorum; Pyrenophora-Arten, wie beispielsweise Pyrenophora teres oder Pyrenophora tritici repentis; Ramularia-Arten, wie beispielsweise Ramularia collo-cygni oder Ramularia areola; Rhynchosporium-Arten, wie beispielsweise Rhynchosporium secalis; Septoria-Arten, wie beispielsweise Septoria apii oder Septoria lycopersici; Stagonospora-Arten, wie beispielsweise Stagonospora nodorum; Typhula-Arten, wie beispielsweise Typhula incarnata; Venturia-Arten, wie beispielsweise Venturia inaequalis;
Wurzel- und Stängelkrankheiten, hervorgerufen durch z.B. Corticium-Arten, wie beispielsweise Corticium graminearum; Fusarium-Arten, wie beispielsweise Fusarium oxysporum; Gaeumannomyces-Arten, wie beispielsweise Gaeumannomyces graminis; Plasmodiophora-Arten, wie beispielsweise Plasmodiophora brassicae; Rhizoctonia-Arten, wie beispielsweise Rhizoctonia solani; Sarocladium-Arten, wie beispielsweise Sarocladium oryzae; Sclerotium-Arten, wie beispielsweise Sclerotium oryzae; Tapesia-Arten, wie beispielsweise Tapesia acuformis; Thielaviopsis-Arten, wie beispielsweise Thielaviopsis basicola;
Ähren- und Rispenerkrankungen (inklusive Maiskolben), hervorgerufen durch z.B. Alternaria-Arten, wie beispielsweise Alternaria spp.; Aspergillus-Arten, wie beispielsweise Aspergillus flavus; Cladosporium-Arten, wie beispielsweise Cladosporium cladosporioides; Claviceps-Arten, wie beispielsweise Claviceps purpurea; Fusarium-Arten, wie beispielsweise Fusarium culmorum; Gibberella-Arten, wie beispielsweise Gibberella zeae; Monographella-Arten, wie beispielsweise Monographella nivalis; Stagonospora-Arten, wie beispielsweise Stagonospora nodorum;
Erkrankungen, hervorgerufen durch Brandpilze wie z.B. Sphacelotheca-Arten, wie beispielsweise Sphacelotheca reiliana; Tilletia-Arten, wie beispielsweise Tilletia caries oder Tilletia controversa; Urocystis-Arten, wie beispielsweise Urocystis occulta; Ustilago-Arten, wie beispielsweise Ustilago nuda;
Fruchtfäule hervorgerufen durch z.B. Aspergillus-Arten, wie beispielsweise Aspergillus flavus; Botrytis-Arten, wie beispielsweise Botrytis cinerea; Penicillium-Arten, wie beispielsweise Penicillium expansum oder Penicillium purpurogenum; Rhizopus -Arten, wie beispielsweise Rhizopus stolonifer; Sclerotinia-Arten, wie beispielsweise Sclerotinia sclerotiorum; Verticilium-Arten, wie beispielsweise Verticilium alboatrum;
Samen- und bodenbürtige Fäulen und Welken, sowie Sämlingserkrankungen, hervorgerufen durch z.B. Alternaria-Arten, wie beispielsweise Alternaria brassicicola; Aphanomyces-Arten, wie beispielsweise Aphanomyces euteiches; Ascochyta-Arten, wie beispielsweise Ascochyta lentis; Aspergillus-Arten, wie beispielsweise Aspergillus flavus; Cladosporium-Arten, wie beispielsweise Cladosporium herbarum; Cochliobolus-Arten, wie beispielsweise Cochliobolus sativus (Konidienform: Drechslera, Bipolaris Syn: Helminthosporium); Colletotrichum-Arten, wie beispielsweise Colletotrichum coccodes; Fusarium-Arten, wie beispielsweise Fusarium culmorum; Gibberella-Arten, wie beispielsweise Gibberella zeae; Macrophomina-Arten, wie beispielsweise Macrophomina phaseolina; Microdochium-Arten, wie beispielsweise Microdochium nivale; Monographella-Arten, wie beispielsweise Monographella nivalis; Penicillium-Arten, wie beispielsweise Penicillium expansum; Phoma-Arten, wie beispielsweise Phoma lingam; Phomopsis-Arten, wie beispielsweise Phomopsis sojae; Phytophthora Arten, wie beispielsweise Phytophthora cactorum; Pyrenophora-Arten, wie beispielsweise Pyrenophora graminea; Pyricularia-Arten, wie beispielsweise Pyricularia oryzae; Pythium-Arten, wie beispielsweise Pythium ultimum; Rhizoctonia-Arten, wie beispielsweise Rhizoctonia solani; Rhizopus-Arten, wie beispielsweise Rhizopus oryzae; Sclerotium-Arten, wie beispielsweise Sclerotium rolfsii; Septoria-Arten, wie beispielsweise Septoria nodorum; Typhula-Arten, wie beispielsweise Typhula incarnata; Verticillium-Arten, wie beispielsweise Verticillium dahliae;
Krebserkrankungen, Gallen und Hexenbesen, hervorgerufen durch z.B. Nectria-Arten, wie beispielsweise Nectria galligena;
Welkeerkrankungen hervorgerufen durch z.B. Monilinia-Arten, wie beispielsweise Monilinia laxa;
Deformationen von Blättern, Blüten und Früchten, hervorgerufen durch z.B. Exobasidium-Arten, wie beispielsweise Exobasidium vexans; Taphrina-Arten, wie beispielsweise Taphrina deformans;
Degenerationserkrankungen holziger pflanzen, hervorgerufen durch z.B. Esca-Arten, wie beispielsweise Phaeomoniella chlamydospora, Phaeoacremonium aleophilum oder Fomitiporia mediterranea; Ganoderma-Arten, wie beispielsweise Ganoderma boninense;
Blüten- und Samenerkrankungen, hervorgerufen durch z.B. Botrytis-Arten, wie beispielsweise Botrytis cinerea;
Erkrankungen von Pflanzenknollen, hervorgerufen durch z.B. Rhizoctonia-Arten, wie beispielsweise Rhizoctonia solani; Helminthosporium-Arten, wie beispielsweise Helminthosporium solani;
Der erfindungsgemäße Wirkstoff weist auch eine sehr gute stärkende Wirkung in Pflanzen auf. Er eignet sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze und Bakterien zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lässt sich der erfindungsgemäße Wirkstoff mit besonders gutem Erfolg zur Bekämpfung von Krankheiten im Wein-, Obst-, Kartoffel- und Gemüseanbau, wie beispielsweise insbesondere gegen falsche Mehltaupilze, Oomyceten, wie beispielsweise Phytophthora-, Plasmopara-, Pseudoperonospora- und Pythium-Arten, einsetzen.

Der erfindungsgemäße Wirkstoff eignen sich auch zur Steigerung des Ernteertrages. Er ist außerdem mindertoxisch und weist eine gute Pflanzenverträglichkeit auf.

Die erfindungsgemäße Verbindung kann gegebenenfalls in bestimmten Konzentrationen bzw. Aufwandmengen auch als Herbizid, Safener, Wachstumsregulator oder Mittel zur Verbesserung der Pflanzeneigenschaften, oder als Mikrobizid, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Er lässt sich gegebenenfalls auch als Insektizid verwenden. Er lässt sich gegebenenfalls auch als Zwischen- oder Vorprodukte für die Synthese weiterer Wirkstoffe einsetzen.

Die erfindungsgemäße Behandlung der Pflanzen und Pflanzenteile mit den Wirkstoffen bzw. Mitteln erfolgt direkt oder durch Einwirkung auf deren Umgebung, Lebensraum oder Lagerraum nach den üblichen Behandlungsmethoden, z.B. durch Tauchen, (Ver-)Spritzen, (Ver-)Sprühen, Berieseln, Verdampfen, Zerstäuben, Vernebeln, (Ver-)Streuen, Verschäumen, Bestreichen, Verstreichen, Gießen (drenchen), Tröpfchenbewässerung und bei Vermehrungsmaterial, insbesondere bei Samen, weiterhin durch Trockenbeizen, Nassbeizen, Schlämmbeizen, Inkrustieren, ein- oder mehrschichtiges Umhüllen usw. Es ist ferner möglich, die Wirkstoffe nach dem Ultra-Low-Volume-Verfahren auszubringen oder die Wirkstoffzubereitung oder den Wirkstoff selbst in den Boden zu injizieren.

Der erfindungsgemäße Wirkstoff bzw. Mittel kann außerdem im Materialschutz zum Schutz von technischen Materialien gegen Befall und Zerstörung durch unerwünschte Mikroorganismen, wie z.B. Pilzen, eingesetzt werden.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz. Die erfindungsgemäßen Wirkstoffe bzw. Mittel können nachteilige Effekte wie Vermodern, Verfall, Ver-, Entfärbung oder Verschimmeln verhindern.

Das erfindungsgemäße Verfahren zum Bekämpfen von unerwünschten Pilzen kann auch zum Schutz von so genannte Storage Goods verwendet werden. Unter "Storage Goods" werden dabei natürliche Substanzen pflanzlichen oder tierischen Ursprungs oder deren Verarbeitungsprodukte, welche der Natur entnommen wurden und für die Langzeitschutz gewünscht ist, verstanden. Storage Goods pflanzlichen Ursprungs, wie z.B. Pflanzen oder Pflanzenteile, wie Stiele, Blätter, Knollen, Samen, Früchte, Körner, können in frisch geerntetem Zustand oder nach Verarbeitung durch (Vor-)Trocknen, Befeuchten, Zerkleinern, Mahlen, Pressen oder Rösten, geschützt werden. Storage Goods umfasst auch Nutzholz, sei es unverarbeitet, wie Bauholz, Stromleitungsmasten und Schranken, oder in Form fertiger Produkte, wie Möbel. Storage Goods tierischen Ursprungs sind beispielsweise Felle, Leder, Pelze und Haare. Die erfindungsgemäßen Wirkstoffe können nachteilige Effekte wie Vermodern, Verfall, Ver-, Entfärbung oder Verschimmeln verhindern.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, Holz verfärbende und Holz zerstörende Pilze (Basidiomyceten) sowie gegen Schleimorganismen und Algen. Es seien beispielsweise Mikroorganismen der folgenden Gattungen genannt: Alternaria, wie Alternaria tenuis; Aspergillus, wie Aspergillus niger; Chaetomium, wie Chaetomium globosum; Coniophora, wie Coniophora puetana; Lentinus, wie Lentinus tigrinus; Penicillium, wie Penicillium glaucum; Polyporus, wie Polyporus versicolor; Aureobasidium, wie Aureobasidium pullulans; Sclerophoma, wie Sclerophoma pityophila; Trichoderma, wie Trichoderma viride; Escherichia, wie Escherichia coli; Pseudomonas, wie Pseudomonas aeruginosa; Staphylococcus, wie Staphylococcus aureus.

Die erfindungsgemäßen fungiziden Mittel können zur Bekämpfung von phytopathogenen Pilzen kurativ oder protektiv eingesetzt werden. Die Erfindung betrifft daher auch kurative und protektive Verfahren zum Bekämpfen von phytopathogenen Pilzen durch die Verwendung der erfindungsgemäßen Wirkstoffe oder Mittel, welche auf das Saatgut, die Pflanze oder Pflanzenteile, die Früchten oder den Boden, in welcher die Pflanzen wachsen, ausgebracht wird.

Die erfindungsgemäßen Mittel zum Bekämpfen von phytopathogenen Pilzen im Pflanzenschutz umfassen eine wirksame, aber nicht-phytotoxische Menge der erfindungsgemäßen Wirkstoffe. "Wirksame, aber nicht-phytotoxische Menge" bedeutet eine Menge des erfindungsgemäßen Mittels, die ausreichend ist, um die Pilzerkrankung der Pflanze ausreichend zu kontrollieren oder ganz abzutöten und die gleichzeitig keine nennenswerten Symptome von Phytotoxizität mit sich bringt. Diese Aufwandmenge kann im Allgemeinen in einem größeren Bereich variieren. Sie hängt von mehreren Faktoren ab, z.B. vom zu bekämpfenden Pilz, der Pflanze, den klimatischen Verhältnissen und den Inhaltsstoffen der erfindungsgemäßen Mittel.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Als Pflanzen, welche erfindungsgemäß behandelt werden können, seien folgende erwähnt: Baumwolle, Flachs, Weinrebe, Obst, Gemüse, wie *Rosaceae sp.* (beispielsweise Kernfrüchte wie Apfel und Birne, aber auch Steinfrüchte wie Aprikosen, Kirschen, Mandeln und Pfirsiche und Beerenfrüchte wie Erdbeeren), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (beispielsweise Bananenbäume und -plantagen), *Rubiaceae sp.* (beispielsweise Kaffee), *Theaceae sp., Sterculiceae sp., Rutaceae sp.* (beispielsweise Zitronen, Organen und Grapefruit); *Solanaceae sp.* (beispielsweise Tomaten), *Liliaceae sp., Asteraceae sp.* (beispielsweise Salat), *Umbelliferae sp., Cruciferae sp., Chenopodiaceae sp., Cucurbitaceae sp.* (beispielsweise Gurke), *Alliaceae sp.* (beispielsweise Lauch, Zwiebel), *Papilionaceae sp.* (beispielsweise Erbsen); Hauptnutzpflanzen, wie *Gramineae sp.* (beispielsweise Mais, Rasen, Getreide wie Weizen, Roggen, Reis, Gerste, Hafer, Hirse und Triticale), *Asteraceae sp.* (beispielsweise Sonnenblume), *Brassicaceae sp.* (beispielsweise Weißkohl, Rotkohl, Brokkoli, Blumenkohl, Rosenkohl, Pak Choi, Kohlrabi, Radieschen sowie Raps, Senf, Meerrettich und Kresse), *Fabacae sp.* (beispielsweise Bohne, Erdnüsse), *Papilionaceae sp.* (beispielsweise Sojabohne), *Solanaceae sp.* (beispielsweise Kartoffeln), *Chenopodiaceae sp.* (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen.

Es hat sich herausgestellt, dass Mischungen enthaltend
(A) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat in der neuen kristallinen Form und
(B) mindestens ein Fungizid ausgwählt aus der Liste (siehe unten),
nicht nur ein erwartungsgemäß additiv erweiteretes Spektrums zeigen, sondern darüberhinaus auch synergistisch wirken. Überraschenderweise ist dieser synergistische Effekt stärker ausgeprägt als bei der Mischung von amorphem 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat in Kombination mit Fungiziden (WO2013/127704).

Als Mischpartner (B) eignen sich insbesondere die folgenden Fungizide:
(1) Inhibitoren der Ergosterol-Biosynthese, wie beispielsweise (1.1) Aldimorph, (1.2) Azaconazol, (1.3) Bitertanol, (1.4) Bromuconazol, (1.5) Cyproconazol, (1.6) Diclobutrazol, (1.7) Difenoconazol, (1.8) Diniconazol, (1.9) Diniconazol-M, (1.10) Dodemorph, (1.11) Dodemorph Acetat, (1.12) Epoxiconazol, (1.13) Etaconazol, (1.14) Fenarimol, (1.15) Fenbuconazol, (1.16) Fenhexamid, (1.17) Fenpropidin, (1.18) Fenpropimorph, (1.19) Fluquinconazol, (1.20) Flurprimidol, (1.21) Flusilazol, (1.22) Flutriafol, (1.23) Furconazol, (1.24) Furconazol-Cis, (1.25) Hexaconazol, (1.26) Imazalil, (1.27) Imazalil Sulfat, (1.28) Imibenconazol, (1.29) Ipconazol, (1.30) Metconazol, (1.31) Myclobutanil, (1.32) Naftifin, (1.33) Nuarimol, (1.34) Oxpoconazol, (1.35) Paclobutrazol, (1.36) Pefurazoat, (1.37) Penconazol, (1.38) Piperalin, (1.39) Prochloraz, (1.40) Propiconazol, (1.41) Prothioconazol, (1.42) Pyributicarb, (1.43) Pyrifenox, (1.44) Quinconazol, (1.45) Simeconazol, (1.46) Spiroxamin, (1.47) Tebuconazol, (1.48) Terbinafin, (1.49) Tetraconazol, (1.50) Triadimefon, (1.51) Triadimenol, (1.52) Tridemorph, (1.53) Triflumizol, (1.54) Triforin, (1.55) Triticonazol, (1.56) Uniconazol, (1.57) Uniconazol-p, (1.58) Viniconazol, (1.59) Voriconazol, (1.60) 1-(4-Chlorphenyl)-2-(1H-1,2,4-triazol-1-yl)cycloheptanol, (1.61) Methyl-1-(2,2-dimethyl-2,3-dihydro-1H-inden-1-yl)-1H-imidazol-5-carboxylat, (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'- {2-methyl-5-(trifluormethyl)-4-[3-(trimethylsilyl)propoxy]phenyl}imidoformamid und (1.64) O-[1-(4-Methoxyphenoxy)-3,3-dimethylbutan-2-yl]-1H-imidazol-1-carbothioat, (1.65) Pyrisoxazole.
(2) Inhibitoren der Respiration (Atmungsketten-Inhibitoren), wie beispielsweise (2.1) Bixafen, (2.2) Boscalid, (2.3) Carboxin, (2.4) Diflumetorim, (2.5) Fenfuram, (2.6) Fluopyram, (2.7) Flutolanil, (2.8) Fluxapyroxad, (2.9) Furametpyr, (2.10) Furmecyclox, (2.11) Isopyrazam Mischung des syn-epimeren Razemates 1RS,4SR,9RS und des anti-empimeren Razemates 1RS,4SR,9SR, (2.12) Isopyrazam (anti-epimeres Razemat), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Razemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil, (2.19) Oxycarboxin, (2.20) Penflufen, (2.21) Penthiopyrad, (2.22) Sedaxane, (2.23) Thifluzamid, (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid, (2.28) 5,8-Difluor-N-[2-(2-fluor-4-{[4-(trifluormethyl)pyridin-2-yl]oxy}phenyl)ethyl]quinazolin-4-amin, (2.29) Benzovindiflupyr, (2.30) N-[(1S,4R)-9-(Dichlormethylen)-1,2,3,4-tetrahydro-1,4-methanonaphthalen-5-yl]-3-(difluormethyl)-1-methyl-1H-pyrazol-4-carboxamid und (2.31) N-[(1R,4S)-9-(Dichlormethylen)-1,2,3,4-tetrahydro-1,4-methanonaphthalen-5-yl]-3-(difluormethyl)-1-methyl-1H-pyrazol-4-carboxamid, (2.32) 3-(Difluormethyl)-1-methyl-N-(1,1,3-trimethyl-2,3-dihydro-1H-inden-4-yl)-1H-pyrazol-4-carboxamid, (2.33) 1,3,5-Trimethyl-N-(1,1,3-trimethyl-2,3-dihydro-1H-inden-4-yl)-1H-pyrazol-4-carboxamid, (2.34) 1-Methyl-3-(trifluormethyl)-N-(1,1,3-trimethyl-2,3-dihydro-1H-inden-4-yl)-1H-pyrazol-4-carboxamid, (2.35) 1-Methyl-3-(trifluormethyl)-N-[(3R)-1,1,3-trimethyl-2,3-dihydro-1H-inden-4-yl]-1H-pyrazol-4-carboxamid, (2.36) 1-Methyl-3-(trifluormethyl)-N-[(3S)-1,1,3-trimethyl-2,3-dihydro-1H-inden-4-yl]-1H-pyrazol-4-carboxamid, (2.37) 3-(Difluormethyl)-1-methyl-N-[(3S)-1,1,3-trimethyl-2,3-dihydro-1H-inden-4-yl]-1H-pyrazol-4-carboxamid, (2.38) 3-(Difluormethyl)-1-methyl-N-[(3R)-1,1,3-trimethyl-2,3-dihydro-1H-inden-4-yl]-1H-pyrazol-4-carboxamid, (2.39) 1,3,5-Trimethyl-N-[(3R)-1,1,3-trimethyl-2,3-dihydro-1H-inden-4-yl]-1H-pyrazol-4-carboxamid, (2.40) 1,3,5-Trimethyl-N-[(3S)-1,1,3-trimethyl-2,3-dihydro-1H-inden-4-yl]-1H-pyrazol-4-carboxamid, (2.41) Benodanil, (2.42) 2-Chlor-N-(1,1,3-trimethyl-2,3-dihydro-1H-inden-4-yl)pyridine-3-carboxamid, (2.43) Isofetamid
(3) Inhibitoren der Respiration (Atmungsketten-Inhibitoren) am Komplex III der Atumungskette, wie beispielsweise (3.1) Ametoctradin, (3.2) Amisulbrom, (3.3) Azoxystrobin, (3.4) Cyazofamid, (3.5) Coumethoxystrobin, (3.6) Coumoxystrobin, (3.5) Dimoxystrobin, (3.8) Enestroburin, (3.9) Famoxadon, (3.10) Fenamidon, (3.11) Flufenoxystrobin, (3.12) Fluoxastrobin, (3.13) Kresoxim-Methyl, (3.14) Metominostrobin, (3.15) Orysastrobin, (3.16) Picoxystrobin, (3.17) Pyraclostrobin, (3.18) Pyrametostrobin, (3.19) Pyraoxystrobin, (3.20) Pyribencarb, (3.21) Triclopyricarb, (3.22) Trifloxystrobin, (3.23) (2E)-2-(2-{[6-(3-Chlor-2-methylphenoxy)-5-fluorpyrimidin-4-yl]oxy}phenyl)-2-(methoxyimino)-N-methylethanamid, (3.24) (2E)-2-(Methoxyimino)-N-methyl-2-(2-{[({(1E)-1-[3-(trifluormethyl)phenyl]ethyliden}amino)oxy]methyl}phenyl)ethanamid, (3.25) (2E)-2-(Methoxyimino)-N-methyl-2-{2-[(E)-({1-[3-(trifluormethyl)phenyl]ethoxy}imino)methyl]phenyl}ethanamid, (3.26) (2E)-2-{2-[({[(1E)-1-(3-{[(E)-1-Fluor-2-phenylethenyl]oxy}phenyl)ethyliden]amino}oxy)methyl]phenyl}-2-(methoxyimino)-N-methylethanamid, (3.27) (2E)-2-{2-[({[(2E,3E)-4-(2,6-Dichlorphenyl)but-3-en-2-yliden]amino}oxy)methyl]phenyl}-2-(methoxyimino)-N-methylethanamid, (3.28) 2-Chlor-N-(1,1,3-trimethyl-2,3-dihydro-1H-inden-4-yl)pyridin-3-carboxamid, (3.29) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.30) Methyl-(2E)-2-{2-[({cyclopropyl[(4-methoxyphenyl)imino]methyl}sulfanyl)methyl]phenyl}-3-methoxyprop-2-enoat, (3.31) N-(3-Ethyl-3,5,5-trimethylcyclohexyl)-3-(formylamino)-2-hydroxybenzamid, (3.32) 2-{2-[(2,5-Dimethylphenoxy)methyl]phenyl}-2-methoxy-N-methylacetamid,
(4) Inhibitoren der Mitose und Zellteilung, wie beispielsweise (4.1) Benomyl, (4.2) Carbendazim, (4.3) Chlorfenazol, (4.4) Diethofencarb, (4.5) Ethaboxam, (4.6) Fluopicolid, (4.7) Fuberidazol, (4.8) Pencycuron, (4.9) Thiabendazol, (4.10) Thiophanat-Methyl, (4.11) Thiophanat, (4.12) Zoxamid, (4.13) 5-Chlor-7-(4-methylpiperidin-1-yl)-6-(2,4,6-trifluorphenyl)[1,2,4]triazolo[1,5-a]pyrimidin und (4.14) 3-Chlor-5-(6-chlorpyridin-3-yl)-6-methyl-4-(2,4,6-trifluorphenyl)pyridazin.
(5) Verbindungen mit Multisite-Aktivität, wie beispielsweise (5.1) Bordeauxmischung, (5.2) Captafol, (5.3) Captan, (5.4) Chlorthalonil, (5.5) Kupferzubereitungen wie Kupferhydroxid, (5.6) Kupfernaphthenat, (5.7) Kupferoxid, (5.8) Kupferoxychlorid, (5.9) Kupfersulfat, (5.10) Dichlofluanid, (5.11) Dithianon, (5.12) Dodine, (5.13) Dodine freie Base, (5.14) Ferbam, (5.15) Fluorfolpet, (5.16) Folpet, (5.17) Guazatin, (5.18) Guazatinacetat, (5.19) Iminoctadin, (5.20) Iminoctadinalbesilat, (5.21) Iminoctadintriacetat, (5.22) Mankupfer, (5.23) Mancozeb, (5.24) Maneb, (5.25) Metiram, (5.26) Zinkmetiram, (5.27) Kupfer-Oxin, (5.28) Propamidin, (5.29) Propineb, (5.30) Schwefel und Schwefelzubereitungen wie beispielsweise Calciumpolysulfid, (5.31) Thiram, (5.32) Tolylfluanid, (5.33) Zineb, (5.34) Ziram und (5,35) Anilazin.
(6) Resistenzinduktoren, wie beispielsweise (6.1) Acibenzolar-S-Methyl, (6.2) Isotianil, (6.3) Probenazol, (6.4) Tiadinil und (6.5) Laminarin.
(7) Inhibitoren der Aminosäure- und Protein-Biosynthese, wie beispielsweise (7.1) , (7.2) Blasticidin-S, (7.3) Cyprodinil, (7.4) Kasugamycin, (7.5) Kasugamycin Hydrochlorid Hydrat, (7.6) Mepanipyrim, (7.7) Pyrimethanil, (7.8) 3-(5-Fluor-3,3,4,4-tetramethyl-3,4-dihydroisochinolin-1-yl)chinolin und (7.9) Oxytetracyclin und (7.10) Streptomycin.
(8) Inhibitoren der ATP Produktion, wie beispielsweise (8.1) Fentin Acetat, (8.2) Fentin Chlorid, (8.3) Fentin Hydroxid und (8.4) Silthiofam.
(9) Inhibitoren der Zellwandsynthese, wie beispielsweise (9.1) Benthiavalicarb, (9.2) Dimethomorph, (9.3) Flumorph, (9.4) Iprovalicarb, (9.5) Mandipropamid, (9.6) Polyoxins, (9.7) Polyoxorim, (9.8) Validamycin A, (9.9) Valifenalat und (9.10) Polyoxin B.
(10) Inhibitoren der Lipid- und Membran-Synthese, wie beispielsweise (10.1) Biphenyl, (10.2) Chlorneb, (10.3) Dicloran, (10.4) Edifenphos, (10.5) Etridiazol, (10.6) Iodocarb, (10.7) Iprobenfos, (10.8) Isoprothiolan, (10.9) Propamocarb, (10.10) Propamocarb Hydrochlorid, (10.11) Prothiocarb" (10.12) Pyrazophos, (10.13) Quintozen, (10.14) Tecnazene und (10.15) Tolclofos-Methyl.
(11) Inhibitoren der Melanin-Biosynthese, wie beispielsweise (11.1) Carpropamid, (11.2) Diclocymet, (11.3) Fenoxanil, (11.4) Fthalid, (11.5) Pyroquilon, (11.6) Tricyclazol, und (11.7) 2,2,2-Trifluorethyl {3-methyl-1-[(4-methylbenzoyl)amino]butan-2-yl}carbamat.
(12) Inhibitoren der Nukleinsäuresynthese, wie beispielsweise (12.1) Benalaxyl, (12.2) Benalaxyl-M (Kiralaxyl), (12.3) Bupirimat, (12.4) Clozylacon, (12.5) Dimethirimol, (12.6) Ethirimol, (12.7) Furalaxyl, (12.8) Hymexazol, (12.9) Metalaxyl, (12.10) Metalaxyl-M (Mefenoxam), (12.11) Ofurace, (12.12) Oxadixyl, (12.13) Oxolinsäure und (12.14) Octhilinon.
(13) Inhibitoren der Signaltransduktion, wie beispielsweise (13.1) Chlozolinat, (13.2) Fenpiclonil, (13.3) Fludioxonil, (13.4) Iprodion, (13.5) Procymidon, (13.6) Quinoxyfen, (13.7) Vinclozolin und (13.8) Proquinazid.
(14) Entkoppler, wie beispielsweise (14.1) Binapacryl, (14.2) Dinocap, (14.3) Ferimzon, (14.4) Fluazinam und (14.5) Meptyldinocap.
(15) Weitere Verbindungen, wie beispielsweise (15.1) Benthiazol, (15.2) Bethoxazin, (15.3) Capsimycin, (15.4) Carvon, (15.5) Chinomethionat, (15.6) Pyriofenon (Chlazafenon), (15.7) Cufraneb, (15.8) Cyflufenamid, (15.9) Cymoxanil, (15.10) Cyprosulfamid, (15.11) Dazomet, (15.12) Debacarb, (15.13) Dichlorphen, (15.14) Diclomezin, (15.15) Difenzoquat, (15.16) Difenzoquat Methylsulphat, (15.17) Diphenylamin, (15.18) Ecomat, (15.19) Fenpyrazamin, (15.20) Flumetover, (15.21) Fluorimid, (15.22) Flusulfamid, (15.23) Flutianil, (15.24) Fosetyl-Aluminium, (15.25) Fosetyl-Calcium, (15.26) Fosetyl-Natrium, (15.27) Hexachlorbenzol, (15.28) Irumamycin, (15.29) Methasulfocarb, (15.30) Methylisothiocyanat, (15.31) Metrafenon, (15.32) Mildiomycin, (15.33) Natamycin, (15.34) Nickel Dimethyldithiocarbamat, (15.35) Nitrothal-Isopropyl, (15.36) Octhilinone, (15.37) Oxamocarb, (15.38) Oxyfenthiin, (15.39) Pentachlorphenol und dessen Salze, (15.40) Phenothrin, (15.41) Phosphorsäure und deren Salze, (15.42) Propamocarb-Fosetylat, (15.43) Propanosin-Natrium, (15.44) Pyrimorph, (15.45) (2E)-3-(4-Tert-butylphenyl)-3-(2-chlorpyridin-4-yl)-1-(morpholin-4-yl)prop-2-en-1-on, (15.46) (2Z)-3-(4-Tert-butylphenyl)-3-(2-chlorpyridin-4-yl)-1-(morpholin-4-yl)prop-2-en-1-on, (15.47) Pyrrolnitrin, (15.48) Tebufloquin, (15.49) Tecloftalam, (15.50) Tolnifanide, (15.51) Triazoxid, (15.52) Trichlamid, (15.53) Zarilamid, (15.54) (3S,6S,7R,8R)-8-Benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoat, (15.55) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.56) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.57) 1-(4-{4-[5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.58) 1-(4-Methoxyphenoxy)-3,3-dimethylbutan-2-yl 1H-imidazole-1-carboxylat, (15.59) 2,3,5,6-Tetrachlor-4-(methylsulfonyl)pyridin, (15.60) 2,3-Dibutyl-6-chlorthieno[2,3-d]pyrimidin-4(3H)-on, (15.61) 2,6-Dimethyl-1H,5H-[1,4]dithiino[2,3-c:5,6-c']dipyrrole-1,3,5,7(2H,6H)-tetron, (15.62) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, (15.63) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, (15.64) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, (15.65) 2-Butoxy-6-iodo-3-propyl-4H-chromen-4-on, (15.66) 2-Chlor-5-[2-chlor-1-(2,6-difluor-4-methoxyphenyl)-4-methyl-1H-imidazol-5-yl]pyridin, (15.67) 2-Phenylphenol und Salze, (15.68) 3-(4,4,5-Trifluor-3,3-dimethyl-3,4-dihydroisochinolin-1-yl)chinolin, (15.69) 3,4,5-Trichlorpyridine-2,6-dicarbonitril, (15.70) 3-Chlor-5-(4-chlorphenyl)-4-(2,6-difluorphenyl)-6-methylpyridazin, (15.71) 4-(4-Chlorphenyl)-5-(2,6-difluorphenyl)-3,6-dimethylpyridazin, (15.72) 5-Amino-1,3,4-thiadiazole-2-thiol, (15.73) 5-Chlor-N'-phenyl-N'-(prop-2-yn-1-yl)thiophene-2-sulfonohydrazid, (15.74) 5-Fluor-2-[(4-fluorbenzyl)oxy]pyrimidin-4-amin, (15.75) 5-Fluor-2-[(4-methylbenzyl)oxy]pyrimidin-4-amin, (15.76) 5-Methyl-6-octyl[1,2,4]triazolo[1,5-a]pyrimidin-7-amin, (15.77) Ethyl (2Z)-3-amino-2-cyano-3-phenylacrylat, (15.78) N'-(4-{[3-(4-Chlorbenzyl)-1,2,4-thiadiazol-5-yl]oxy}-2,5-dimethylphenyl)-N-ethyl-N-methylimidoformamid, (15.79) N-(4-Chlorbenzyl)-3-[3-methoxy-4-(prop-2-yn-1-yloxy)phenyl]propanamid, (15.80) N-[(4-Chlorphenyl)(cyano)methyl]-3-[3-methoxy-4-(prop-2-yn-1-yloxy)phenyl]propanamid, (15.81) N-[(5-Brom-3-chlorpyridin-2-yl)methyl]-2,4-dichlornicotinamid, (15.82) N-[1-(5-Brom-3-chlorpyridin-2-yl)ethyl]-2,4-dichlornicotinamid, (15.83) N-[1-(5-Brom-3-chlorpyridin-2-yl)ethyl]-2-fluor-4-iodonicotinamid, (15.84) N-{(E)-[(Cyclopropylmethoxy)imino][6-(difluormethoxy)-2,3-difluorphenyl]methyl}-2-phenylacetamid, (15.85) N-{(Z)-[(Cyclopropylmethoxy)imino][6-(difluormethoxy)-2,3-difluorphenyl]methyl}-2-phenylacetamid, (15.86) N'-{4-[(3-Tert-butyl-4-cyano-1,2-thiazol-5-yl)oxy]-2-chlor-5-methylphenyl}-N-ethyl-N-methylimidoformamid, (15.87) N-Methyl-2-(1-{[5-methyl-3-(trifluormethyl)-1H-pyrazol-1-yl]acetyl}piperidin-4-yl)-N-(1,2,3,4-tetrahydronaphthalen-1-yl)-1,3-thiazole-4-carboxamid, (15.88) N-Methyl-2-(1-{[5-methyl-3-(trifluormethyl)-1H-pyrazol-1-yl]acetyl}piperidin-4-yl)-N-[(1R)-1,2,3,4-tetrahydronaphthalen-1-yl]-1,3-thiazole-4-carboxamid, (15.89) N-Methyl-2-(1-{[5-methyl-3-(trifluormethyl)-1H-pyrazol-1-yl]acetyl}piperidin-4-yl)-N-[(1S)-1,2,3,4-tetrahydronaphthalen-1-yl]-1,3-thiazole-4-carboxamid, (15.90) Pentyl {6-[({[(1-methyl-1H-tetrazol-5-yl)(phenyl)methylene]amino}oxy)methyl]pyridin-2-yl}carbamat, (15.91) Phenazine-1-carbonsäure, (15.92) Chinolin-8-ol, (15.93) Chinolin-8-ol sulfate (2:1), (15.94) Tert-butyl {6-[({[(1-methyl-1H-tetrazol-5-yl)(phenyl)methylene]amino}oxy)methyl]pyridin-2-yl}carbamat, (15.95) 1-Methyl-3-(trifluormethyl)-N-[2'-(trifluormethyl)biphenyl-2-yl]-1H-pyrazol-4-carboxamid, (15.96) N-(4'-Chlorbiphenyl-2-yl)-3-(difluormethyl)-1-methyl-1H-pyrazol-4-carboxamid, (15.97) N-(2',4'-Dichlorbiphenyl-2-yl)-3-(difluormethyl)-1-methyl-1H-pyrazol-4-carboxamid, (15.98) 3-(Difluormethyl)-1-methyl-N-[4'-(trifluormethyl)biphenyl-2-yl]-1H-pyrazol-4-carboxamid, (15.99) N-(2',5'-Difluorbiphenyl-2-yl)-1-methyl-3-(trifluormethyl)-1H-pyrazol-4-carboxamid, (15.100) 3-(Difluormethyl)-1-methyl-N-[4'-(prop-1-yn-1-yl)biphenyl-2-yl]-1H-pyrazol-4-carboxamid, (15.101) 5-Fluor-1,3-dimethyl-N-[4'-(prop-1-yn-1-yl)biphenyl-2-yl]-1H-pyrazol-4-carboxamid, (15.102) 2-Chlor-N-[4'-(prop-1-yn-1-yl)biphenyl-2-yl]nicotinamid, (15.103) 3-(Difluormethyl)-N-[4'-(3,3-dimethylbut-1-yn-1-yl)biphenyl-2-yl]-1-methyl-1H-pyrazol-4-carboxamid, (15.104) N-[4'-(3,3-dimethylbut-1-yn-1-yl)biphenyl-2-yl]-5-fluor-1,3-dimethyl-1H-pyrazol-4-carboxamid, (15.105) 3-(Difluormethyl)-N-(4'-ethynylbiphenyl-2-yl)-1-methyl-1H-pyrazol-4-carboxamid, (15.106) N-(4'-Ethynylbiphenyl-2-yl)-5-fluor-1,3-dimethyl-1H-pyrazol-4-carboxamid, (15.107) 2-Chlor-N-(4'-ethynylbiphenyl-2-yl)nicotinamid, (15.108) 2-Chlor-N-[4'-(3,3-dimethylbut-1-yn-1-yl)biphenyl-2-yl]nicotinamid, (15.109) 4-(Difluormethyl)-2-methyl-N-[4'-(trifluormethyl)biphenyl-2-yl]-1,3-thiazole-5-carboxamid, (15.110) 5-Fluor-N-[4'-(3-hydroxy-3-methylbut-1-yn-1-yl)biphenyl-2-yl]-1,3-dimethyl-1H-pyrazol-4-carboxamid, (15.111) 2-Chlor-N-[4'-(3-hydroxy-3-methylbut-1-yn-1-yl)biphenyl-2-yl]nicotinamid, (15.112) 3-(Difluormethyl)-N-[4'-(3-methoxy-3-methylbut-1-yn-1-yl)biphenyl-2-yl]-1-methyl-1H-pyrazol-4-carboxamid, (15.113) 5-Fluor-N-[4'-(3-methoxy-3-methylbut-1-yn-1-yl)biphenyl-2-yl]-1,3-dimethyl-1H-pyrazol-4-carboxamid, (15.114) 2-Chlor-N-[4'-(3-methoxy-3-methylbut-1-yn-1-yl)biphenyl-2-yl]nicotinamid, (15.115) (5-Brom-2-methoxy-4-methylpyridin-3-yl)(2,3,4-trimethoxy-6-methylphenyl)methanon, (15.116) N-[2-(4-{[3-(4-Chlorphenyl)prop-2-yn-1-yl]oxy}-3-methoxyphenyl)ethyl]-N2-(methylsulfonyl)valinamid, (15.117) 4-Oxo-4-[(2-phenylethyl)amino]butansäure, (15.118) But-3-yn-1-yl {6-[({[(Z)-(1-methyl-1H-tetrazol-5-yl)(phenyl)methylene]amino}oxy)methyl]pyridin-2-yl}carbamat, (15.119) 4-Amino-5-fluorpyrimidin-2-ol (Tautomere Form: 4-Amino-5-fluorpyrimidin-2(1H)-on), (15.120) Propyl 3,4,5-trihydroxybenzoat, (15.121) 1,3-Dimethyl-N-(1,1,3-trimethyl-2,3-dihydro-1H-inden-4-yl)-1H-pyrazol-4-carboxamid, (15.122) 1,3-Dimethyl-N-[(3R)-1,1,3-trimethyl-2,3-dihydro-1H-inden-4-yl]-1H-pyrazol-4-carboxamid, (15.123) 1,3-Dimethyl-N-[(3S)-1,1,3-trimethyl-2,3-dihydro-1H-inden-4-yl]-1H-pyrazol-4-carboxamid, (15.124) [3-(4-Chlor-2-fluorphenyl)-5-(2,4-difluorphenyl)-1,2-oxazol-4-yl](pyridin-3-yl)methanol, (15.125) (S)-[3-(4-Chlor-2-fluorphenyl)-5-(2,4-difluorphenyl)-1,2-oxazol-4-yl](pyridin-3-yl)methanol, (15.126) (R)-[3-(4-Chlor-2-fluorphenyl)-5-(2,4-difluorphenyl)-1,2-oxazol-4-yl](pyridin-3-yl)methanol, (15.127) 2-{[3-(2-Chlorphenyl)-2-(2,4-difluorphenyl)oxiran-2-yl]methyl}-2,4-dihydro-3H-1,2,4-triazol-3-thion, (15.128) 1-{[3-(2-Chlorphenyl)-2-(2,4-difluorphenyl)oxiran-2-yl]methyl}-1H-1,2,4-triazol-5-yl thiocyanat, (15.129) 5-(Allylsulfanyl)-1-{[3-(2-chlorphenyl)-2-(2,4-difluorphenyl)oxiran-2-yl]methyl}-1H-1,2,4-triazol, (15.130) 2-[1-(2,4-Dichlorphenyl)-5-hydroxy-2,6,6-trimethylheptan-4-yl]-2,4-dihydro-3H-1,2,4-triazol-3-thion, (15.131) 2-{[rel(2R,3S)-3-(2-Chlorphenyl)-2-(2,4-difluorphenyl)oxiran-2-yl]methyl}-2,4-dihydro-3H-1,2,4-triazol-3-thion, (15.132) 2-{[rel(2R,3R)-3-(2-Chlorphenyl)-2-(2,4-difluorphenyl)oxiran-2-yl]methyl}-2,4-dihydro-3H-1,2,4-triazol-3-thion, (15.133) 1-{[rel(2R,3S)-3-(2-Chlorphenyl)-2-(2,4-difluorphenyl)oxiran-2-yl]methyl}-1H-1,2,4-triazol-5-yl thiocyanat, (15.134) 1-{[rel(2R,3R)-3-(2-Chlorphenyl)-2-(2,4-difluorphenyl)oxiran-2-yl]methyl}-1H-1,2,4-triazol-5-yl thiocyanat, (15.135) 5-(Allylsulfanyl)-1-{[rel(2R,3S)-3-(2-chlorphenyl)-2-(2,4-difluorphenyl)oxiran-2-yl]methyl}-1H-1,2,4-triazol, (15.136) 5-(Allylsulfanyl)-1-{[rel(2R,3R)-3-(2-chlorphenyl)-2-(2,4-difluorphenyl)oxiran-2-yl]methyl}-1H-1,2,4-triazol, (15.137) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (15.138) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (15.139) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (15.140) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (15.141) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (15.142) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (15.143) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (15.144) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (15.145) 2-Fluor-6-(trifluormethyl)-N-(1,1,3-trimethyl-2,3-dihydro-1H-inden-4-yl)benzamid, (15.146) 2-(6-Benzylpyridin-2-yl)quinazolin, (15.147) 2-[6-(3-Fluor-4-methoxyphenyl)-5-methylpyridin-2-yl]quinazolin, (15.148) 3-(4,4-Difluor-3,3-dimethyl-3,4-dihydroisochinolin-1-yl)chinolin, (15.149) Abscisinsäure, (15.150) 3-(Difluormethyl)-N-methoxy-1-methyl-N-[1-(2,4,6-trichlorphenyl)propan-2-yl]-1H-pyrazol-4-carboxamid, (15.151) N'-[5-Brom-6-(2,3-dihydro-1H-inden-2-yloxy)-2-methylpyridin-3-yl]-N-ethyl-N-methylimidoformamid, (15.152) N'-{5-Brom-6-[1-(3,5-difluorphenyl)ethoxy]-2-methylpyridin-3-yl}-N-ethyl-N-methylimidoformamid, (15.153) N'-{5-Brom-6-[(1R)-1-(3,5-difluorphenyl)ethoxy]-2-methylpyridin-3-yl}-N-ethyl-N-methylimidoformamid, (15.154) N'-{5-Brom-6-[(1S)-1-(3,5-difluorphenyl)ethoxy]-2-methylpyridin-3-yl}-N-ethyl-N-methylimidoformamid, (15.155) N'-{5-Brom-6-[(cis-4-isopropylcyclohexyl)oxy]-2-methylpyridin-3-yl}-N-ethyl-N-methylimidoformamid, (15.156) N'-{5-Brom-6-[(trans-4-isopropylcyclohexyl)oxy]-2-methylpyridin-3-yl}-N-ethyl-N-methylimidoformamid, (15.157) N-Cyclopropyl-3-(difluormethyl)-5-fluor-N-(2-isopropylbenzyl)-1-methyl-1H-pyrazol-4-carboxamid, (15.158) N-Cyclopropyl-N-(2-cyclopropylbenzyl)-3-(difluormethyl)-5-fluor-1-methyl-1H-pyrazol-4-carboxamid, (15.159) N-(2-Tert-butylbenzyl)-N-cyclopropyl-3-(difluormethyl)-5-fluor-1-methyl-1H-pyrazol-4-carboxamid, (15.160) N-(5-Chlor-2-ethylbenzyl)-N-cyclopropyl-3-(difluormethyl)-5-fluor-1-methyl-1H-pyrazol-4-carboxamid, (15.161) N-(5-Chlor-2-isopropylbenzyl)-N-cyclopropyl-3-(difluormethyl)-5-fluor-1-methyl-1H-pyrazol-4-carboxamid, (15.162) N-Cyclopropyl-3-(difluormethyl)-N-(2-ethyl-5-fluorbenzyl)-5-fluor-1-methyl-1H-pyrazol-4-carboxamid, (15.163) N-Cyclopropyl-3-(difluormethyl)-5-fluor-N-(5-fluor-2-isopropylbenzyl)-1-methyl-1H-pyrazol-4-carboxamid, (15.164) N-Cyclopropyl-N-(2-cyclopropyl-5-fluorbenzyl)-3-(difluormethyl)-5-fluor-1-methyl-1H-pyrazol-4-carboxamid, (15.165) N-(2-Cyclopentyl-5-fluorbenzyl)-N-cyclopropyl-3-(difluormethyl)-5-fluor-1-methyl-1H-pyrazol-4-carboxamid, (15.166) N-Cyclopropyl-3-(difluormethyl)-5-fluor-N-(2-fluor-6-isopropylbenzyl)-1-methyl-1H-pyrazol-4-carboxamid, (15.167) N-Cyclopropyl-3-(difluormethyl)-N-(2-ethyl-5-methylbenzyl)-5-fluor-1-methyl-1H-pyrazol-4-carboxamid, (15.168) N-Cyclopropyl-3-(difluormethyl)-5-fluor-N-(2-isopropyl-5-methylbenzyl)-1-methyl-1H-pyrazol-4-carboxamid, (15.169) N-Cyclopropyl-N-(2-cyclopropyl-5-methylbenzyl)-3-(difluormethyl)-5-fluor-1-methyl-1H-pyrazol-4-carboxamid, (15.170) N-(2-Tert-butyl-5-methylbenzyl)-N-cyclopropyl-3-(difluormethyl)-5-fluor-1-methyl-1H-pyrazol-4-carboxamid, (15.171) N-[5-Chlor-2-(trifluormethyl)benzyl]-N-cyclopropyl-3-(difluormethyl)-5-fluor-1-methyl-1H-pyrazol-4-carboxamid, (15.172) N-Cyclopropyl-3-(difluormethyl)-5-fluor-1-methyl-N-[5-methyl-2-(trifluormethyl)benzyl]-1H-pyrazol-4-carboxamid, (15.173) N-[2-Chlor-6-(trifluormethyl)benzyl]-N-cyclopropyl-3-(difluormethyl)-5-fluor-1-methyl-1H-pyrazol-4-carboxamid, (15.174) N-[3-Chlor-2-fluor-6-(trifluormethyl)benzyl]-N-cyclopropyl-3-(difluormethyl)-5-fluor-1-methyl-1H-pyrazol-4-carboxamid, (15.175) N-Cyclopropyl-3-(difluormethyl)-N-(2-ethyl-4,5-dimethylbenzyl)-5-fluor-1-methyl-1H-pyrazol-4-carboxamid, (15.176) N-Cyclopropyl-3-(difluormethyl)-5-fluor-N-(2-isopropylbenzyl)-1-methyl-1H-pyrazol-4-carbothioamid, (15.177) 3-(Difluormethyl)-N-(7-fluor-1,1,3-trimethyl-2,3-dihydro-1H-inden-4-yl)-1-methyl-1H-pyrazol-4-carboxamid, (15.178) 3-(Difluormethyl)-N-[(3R)-7-fluor-1,1,3-trimethyl-2,3-dihydro-1H-inden-4-yl]-1-methyl-1H-pyrazol-4-carboxamid, (15.179) 3-(Difluormethyl)-N-[(3S)-7-fluor-1,1,3-trimethyl-2,3-dihydro-1H-inden-4-yl]-1-methyl-1H-pyrazol-4-carboxamid, (15.180) N'-(2,5-Dimethyl-4-phenoxyphenyl)-N-ethyl-N-methylimidoformamid, (15.181) N'-{4-[(4,5-Dichlor-1,3-thiazol-2-yl)oxy]-2,5-dimethylphenyl}-N-ethyl-N-methylimidoformamid, (15.182) N-(4-Chlor-2,6-difluorphenyl)-4-(2-chlor-4-fluorphenyl)-1,3-dimethyl-1H-pyrazol-5-amin.
   Alle genannten Mischpartner der Klassen (1) bis (15) können, wenn sie auf Grund ihrer funktionellen Gruppen dazu imstande sind, gegebenenfalls mit geeigneten Basen oder Säuren Salze bilden.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Saatgut sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Saatgut.

Die erfindungsgemäße Behandlung der Pflanzen und Pflanzenteile mit dem neuen kristallinen Wirkstoff oder den Wirkstoffkombinationen enthaltend den neuen kristallinen Wirkstoff erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Als Pflanzen, welche erfindungsgemäß behandelt werden können, seien folgende erwähnt: Baumwolle, Flachs, Weinrebe, Obst, Gemüse, wie Rosaceae sp. (beispielsweise Kernfrüchte wie Apfel und Birne, aber auch Steinfrüchte wie Aprikosen, Kirschen, Mandeln und Pfirsiche und Beerenfrüchte wie Erdbeeren), Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp. (beispielsweise Bananenbäume und -plantagen), Rubiaceae sp. (beispielsweise Kaffee), Theaceae sp., Sterculiceae sp., Rutaceae sp. (beispielsweise Zitronen, Organen und Grapefruit); Solanaceae sp. (beispielsweise Tomaten), Liliaceae sp., Asteraceae sp. (beispielsweise Salat), Umbelliferae sp., Cruciferae sp., Chenopodiaceae sp., Cucurbitaceae sp. (beispielsweise Gurke), Alliaceae sp. (beispielsweise Lauch, Zwiebel), Papilionaceae sp. (beispielsweise Erbsen); Hauptnutzpflanzen, wie Gramineae sp. (beispielsweise Mais, Rasen, Getreide wie Weizen, Roggen, Reis, Gerste, Hafer, Hirse und Triticale), Asteraceae sp. (beispielsweise Sonnenblume), Brassicaceae sp. (beispielsweise Weißkohl, Rotkohl, Brokkoli, Blumenkohl, Rosenkohl, Pak Choi, Kohlrabi, Radieschen sowie Raps, Senf, Meerrettich und Kresse), Fabacae sp. (beispielsweise Bohne, Erdnüsse), Papilionaceae sp. (beispielsweise Sojabohne), Solanaceae sp. (beispielsweise Kartoffeln), Chenopodiaceae sp. (beispielsweise Zuckerrübe, Futterrübe, Mangold, Rote Rübe); Nutzpflanzen und Zierpflanzen in Garten und Wald; sowie jeweils genetisch modifizierte Arten dieser Pflanzen.

Die erfindungsgemäße Behandlung der Pflanzen und Pflanzenteile mit dem neuen kristallinen Wirkstoff oder den erfindungsgemäßen Wirkstoffkombinationen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Saatgut, weiterhin durch ein- oder mehrschichtiges Umhüllen.

Ein großer Teil des durch Schädlinge verursachten Schadens an Kulturpflanzen erfolgt bereits durch den Befall des Saatguts während der Lagerung und nach dem Einbringen des Saatguts in den Boden sowie während und unmittelbar nach der Keimung der Pflanzen. Diese Phase ist besonders kritisch, da die Wurzeln und Sprosse der wachsenden Pflanze besonders empfindlich sind und bereits ein geringer Schaden zum Absterben der ganzen Pflanze führen kann. Es besteht daher ein insbesondere großes Interesse daran, das Saatgut und die keimende Pflanze durch den Einsatz geeigneter Mittel zu schützen.

Die vorliegende Erfindung bezieht sich daher insbesondere auch auf ein Verfahren zum Schutz von Saatgut und keimenden Pflanzen vor dem Befall von Schädlingen, indem das Saatgut mit einem erfindungsgemäßen Mittel behandelt wird. Die Erfindung bezieht sich ebenfalls auf die Verwendung der erfindungsgemäßen Mittel zur Behandlung von Saatgut zum Schutz des Saatguts und der daraus entstehenden Pflanze vor Schädlingen. Weiterhin bezieht sich die Erfindung auf Saatgut, welches zum Schutz vor Schädlingen mit einem erfindungsgemäßen Mittel behandelt wurde. Unter Saatgut wird konventionelles Saatgut oder transgenes Saatgut verstanden.

Bei transgenem Saatgut sind die aus diesem Saatgut hervorgehenden Pflanzen zur Expression eines gegen Schädlinge gerichteten Proteins befähigt. Dabei handelt es sich um das Saatgut von Pflanzen, die in der Regel zumindest ein heterologes Gen enthalten, das die Expression eines Polypeptids mit insbesondere insektiziden Eigenschaften steuert. Die heterologen Gene in transgenem Saatgut können dabei aus Mikroorganismen wie Bacillus, Rhizobium, Pseudomonas, Serratia, Trichoderma, Clavibacter, Glomus oder Gliocladium stammen. Die vorliegende Erfindung eignet sich besonders für die Behandlung von transgenem Saatgut, das zumindest ein heterologes Gen enthält, das aus Bacillus sp. stammt und dessen Genprodukt Wirksamkeit gegen Maiszünsler und/oder Maiswurzel-Bohrer zeigt. Besonders bevorzugt handelt es sich dabei um ein heterologes Gen, das aus Bacillus thuringiensis stammt.

Durch die Behandlung solchen Saatguts mit den erfindungsgemäßen Mitteln können bestimmte Schädlinge bereits durch die Expression des z.B. insektiziden Proteins kontrolliert werden, und zusätzlich durch die erfindungsgemäßen Mittel vor Schäden bewahrt werden.

Die erfindungsgemäßen Mittel eignen sich zum Schutz von Saatgut jeglicher Pflanzensorte wie bereits vorstehend genannt, die in der Landwirtschaft, im Gewächshaus, in Forsten oder im Gartenbau eingesetzt wird. Insbesondere handelt es sich dabei um Saatgut von Mais, Erdnuss, Canola, Raps, Mohn, Soja, Baumwolle, Rübe (z.B. Zuckerrübe und Futterrübe), Reis, Hirse, Weizen, Gerste, Hafer, Roggen, Sonnenblume, Tabak, Kartoffeln oder Gemüse (z.B. Tomaten, Kohlgewächs). Die erfindungsgemäßen Mittel eignen sich ebenfalls zur Behandlung des Saatguts von Obstpflanzen und Gemüse wie vorstehend bereits genannt. Besondere Bedeutung kommt der Behandlung des Saatguts von Mais, Soja, Baumwolle, Weizen und Canola oder Raps zu.

Im Rahmen der vorliegenden Erfindung wird das erfindungsgemäßes Mittel alleine oder in einer geeigneten Formulierung auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde.

Im Allgemeinen muss bei der Behandlung des Saatguts darauf geachtet werden, dass die Menge des auf das Saatgut aufgebrachten erfindungsgemäßen Mittels und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die erfindungsgemäßen Mittel können unmittelbar aufgebracht werden, also ohne weitere Komponenten zu enthalten und ohne verdünnt worden zu sein. In der Regel ist es vorzuziehen, die Mittel in Form einer geeigneten Formulierung auf das Saatgut aufzubringen. Geeignete Formulierungen und Verfahren für die Saatgutbehandlung sind dem Fachmann bekannt und werden z.B. in den folgenden Dokumenten beschrieben: US 4,272,417 A, US 4,245,432 A, US 4,808,430 A, US 5,876,739 A, US 2003/0176428 A1, WO 2002/080675 A1, WO 2002/028186 A2.

Die neue kristalline Form von 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat kann in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen. Diese Formulierungen werden in bekannter Weise hergestellt, indem man die Wirkstoffe mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutylnaphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vorzugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, besonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art, auch von Saatgut transgener Pflanzen, eingesetzt werden. Dabei können im Zusammenwirken mit den durch Expression gebildeten Substanzen auch zusätzliche synergistische Effekte auftreten.

Zur Behandlung von Saatgut mit den erfindungsgemäß verwendbaren Beizmittel-Formulierungen oder den daraus durch Zugabe von Wasser hergestellten Zubereitungen kommen alle üblicherweise für die Beizung einsetzbaren Mischgeräte in Betracht. Im einzelnen geht man bei der Beizung so vor, dass man das Saatgut in einen Mischer gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

### Beschreibung der Abbildung 1

Abbildung 1: Röntgen-Pulverdiffraktogramm der neuen kristallinen Form von 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat.

### Herstellung der neuen feste Form

3 g des amorphen 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonats (Form A) (hergestellt wie in WO2012025557 beschrieben) werden mit 40 ml Isopropanol versetzt und auf 82°C erhitzt. Anschließend wird die Suspension 10 Minuten bei dieser Temperatur gerührt. Danach lässt man die Suspension auf 20°C abkühlen und rührt weitere 16 Stunden bei dieser Temperatur. Die ausgefallenen Kristalle werden abgesaugt und bei Raumtemperatur und Normaldruck getrocknet. Man erhält 2,7 g kristallines 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat (Form B) mit einem Schmelzpunkt im Bereich von 153 - 154°C.

Das ¹H-NMR (D₆-DMSO, 298K, 400 MHz) enspricht dem von 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat in amorphem Zustand wie in WO 2012/025557 angegeben.

### Beispiele

Im folgenden wird die Überlegenheit der kristallinen Form über die amorphe Form bei der Bekämpfung von pflanzenpathogenen Schadpilzen beispielhaft dargelegt:

### Beispiel 1

### Phytophthora-Test (Tomate) / protektiv

Zur Herstellung einer zweckmäßigen Wirkstoffzubereitung werden Formulierungen mit Wasser suspendiert und auf die gewünschte Konzentration verdünnt. Im vorliegenden Fall werden sowohl die bekannte amorphe Modifikation der Substanz 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl-methane-sulfonate (Form A), als auch die erfindungsgemäße kristalline Form der Substanz 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl-methanesulfonate (Form B) als 50g a.i./Liter SC-Formulierung verwendet.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von ***Phytophthora infestans*** inokuliert. Die Pflanzen werden dann in einer Inkubationskabine bei ca. 20°C und 100% relativer Luftfeuchtigkeit aufgestellt.

3 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

**Tabelle A**

| **Phytophthora-Test (Tomate) / protektiv** | | | |
|---|---|---|---|
| Substanz | | Aufwandmenge an Wirkstoff in ppm | Wirkungsgrad in% |
| (Form A) | SC-Formulierung mit amorpher Modifikation von 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl-methanesulfonate | 0,2 | 40 |
| | | 0,1 | 15 |
| | | 0,05 | 0 |
| (Form B) | SC-Formulierung mit kristalliner Modifikation von 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl-methanesulfonate | 0,2 | 76 |
| | | 0,1 | 60 |
| | | 0,05 | 40 |

### Beispiel 2

### Plasmopara-Test (Rebe) / protektiv

Zur Herstellung einer zweckmäßigen Wirkstoffzubereitung werden Formulierungen mit Wasser suspendiert und auf die gewünschte Konzentration verdünnt. Im vorliegenden Fall werden sowohl die bekannte amorphe Modifikation der Substanz 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl-methane-sulfonate (Form A), als auch die erfindungsgemäße kristalline Form der Substanz 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl-methanesulfonate (Form B) als 50g a.i./Liter SC-Formulierung verwendet.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wäßrigen Sporensuspension von ***Plasmopara viticola*** inokuliert und verbleiben dann 1 Tag in einer Inkubationskabine bei ca. 20°C und 100% relativer Luftfeuchtigkeit. Anschließend werden die Pflanzen 4 Tage im Gewächshaus bei ca. 21°C und ca. 90% Luftfeuchtigkeit aufgestellt. Die Pflanzen werden dann angefeuchtet und 1 Tag in eine Inkubationskabine gestellt.

6 Tage nach der Inokulation erfolgt die Auswertung. Dabei bedeutet 0% ein Wirkungsgrad, der demjenigen der Kontrolle entspricht, während ein Wirkungsgrad von 100% bedeutet, daß kein Befall beobachtet wird.

**Tabelle B**

| **Plasmopara-Test (Rebe) / protektiv** | | | |
|---|---|---|---|
| Substanz | | Aufwandmenge an Wirkstoff in ppm | Wirkungsgrad in% |
| (Form A) | SC-Formulierung mit amorpher Modifikation von 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl-methanesulfonate | 0,2 | 35 |
| | | 0,1 | 6 |
| | | 0,05 | 0 |
| (Form B) | SC-Formulierung mit kristalliner Modifikation von 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl-methanesulfonate | 0,2 | 75 |
| | | 0,1 | 35 |
| | | 0,05 | 0 |

## Patentansprüche

1. Kristalline Form von 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat, **dadurch gekennzeichnet, dass** ihr Röntgen-Pulverdiffraktogramm bei 25°C mindestens 3 der folgenden 2Θ (2 Theta)-Werte aufweist:
| |
|---|
| 2Θ = 7,75° ± 0,2 |
| 2Θ = 8,89 ± 0,2 |
| 2Θ = 10,91 ± 0,2 |
| 2Θ = 11,45 ± 0,2 |
| 2Θ = 12,72 ± 0,2 |
| 2Θ = 13,46 ± 0,2 |
| 2Θ = 14,54 ± 0,2 |
| 2Θ = 14,75 ± 0,2 |
| 2Θ = 15,53 ± 0,2 |
| 2Θ = 15,90 ± 0,2 |
| 2Θ = 16,33 ± 0,2 |
| 2Θ = 16,65 ± 0,2 |
| 2Θ = 17,49 ± 0,2 |
| 2Θ = 17,71 ± 0,2 |
| 2Θ = 17,89 ± 0,2 |
| 2Θ = 18,27 ± 0,2 |
| 2Θ = 18,47 ± 0,2 |
| 2Θ = 18,86 ± 0,2 |
| 2Θ = 18,99 ± 0,2 |
| 2Θ = 19,18 ± 0,2 |
| 2Θ = 19,63 ± 0,2 |
| 2Θ = 20,03 ± 0,2 |
| 2Θ = 20,22 ± 0,2 |
| 2Θ = 20,53 ± 0,2 |
| 2Θ = 21,11 ± 0,2 |
| 2Θ = 21,32 ± 0,2 |
| 2Θ = 21,50 ± 0,2 |
| 2Θ = 21,84 ± 0,2 |
| 2Θ = 22,01 ± 0,2 |
| 2Θ = 22,28 ± 0,2 |
| 2Θ = 22,61 ± 0,2 |
| 2Θ = 23,05 ± 0,2 |
| 2Θ = 23,39 ± 0,2 |
| 2Θ = 24,02 ± 0,2 |
| 2Θ = 24,22 ± 0,2 |
| 2Θ = 24,62 ± 0,2 |
| 2Θ = 24,97 ± 0,2 |
| 2Θ = 25,59 ± 0,2 |
| 2Θ = 25,99 ± 0,2 |
| 2Θ = 26,31 ± 0,2 |
| 2Θ = 26,67 ± 0,2 |
| 2Θ = 26,85 ± 0,2 |
| 2Θ = 27,07 ± 0,2 |
| 2Θ = 27,43 ± 0,2 |
| 2Θ = 28,21 ± 0,2 |
| 2Θ = 28,34 ± 0,2 |
| 2Θ = 28,78 ± 0,2 |
| 2Θ = 29,59 ± 0,2 |
| 2Θ = 30,19 ± 0,2 |
| 2Θ = 30,37 ± 0,2 |
| 2Θ = 30,96 ± 0,2 |
| 2Θ = 31,29 ± 0,2 |
| 2Θ = 31,70 ± 0,2 |
| 2Θ = 31,81 ± 0,2 |
| 2Θ = 32,16 ± 0,2 |
| 2Θ = 33,02 ± 0,2 |
| 2Θ = 33,39 ± 0,2 |
| 2Θ = 33,64 ± 0,2 |
| 2Θ = 33,79 ± 0,2 |
| 2Θ = 33,80 ± 0,2 |
| 2Θ = 34,20 ± 0,2 |
| 2Θ = 34,77 ± 0,2 |
| 2Θ = 34,89 ± 0,2 |
| 2Θ = 35,66 ± 0,2 |
| 2Θ = 35,95 ± 0,2 |
| 2Θ = 36,67 ± 0,2 |
| 2Θ = 37,03 ± 0,2 |
| 2Θ = 37,38 ± 0,2 |
| 2Θ = 37,51 ± 0,2 |
| 2Θ = 37,87 ± 0,2 |

2. Kristalline Form nach Anspruch 1, **dadurch gekennzeichnet, dass** ihr Röntgenpulverdiffraktogramm bei 25°C mindestend 4 der in Anspruch 1 genannten 2Θ -Werte aufweist.

3. Zusammensetzung umfassend die kristalline Form nach Anspruch 1 oder 2 und geeignete Hilfsstoffe.

4. Verwendung der kristallinen Form nach Anspruch 1 oder 2 oder der Zusammensetzung nach Anspruch 3 zur Herstellung von Mitteln zur Bekämpfung von pflanzenpathogenen Schadpilzen in und/oder auf Pflanzen oder in und/oder auf Saatgut von Pflanzen.

5. Verwendung der kristallinen Form nach Anspruch 1 oder 2 oder der Zusammensetzung nach Anspruch 3 zur Behandlung von Saatgut.

6. Verfahren zur Bekämpfung von pflanzenpathogenen Schadpilzen in und/oder auf Pflanzen oder in und/oder auf Saatgut von Pflanzen, **dadurch gekennzeichnet, dass** die kristalline Form nach Anspruch 1 oder 2 oder die Zusammensetzung nach Anspruch 3 auf die pflanzenpathogenen Schadpilzen und/oder ihren Lebensraum und/oder Saatgut ausgebracht wird.

7. Verfahren zur Herstellung der Verbindung wie in Anspruch 1 oder 2 definiert, das die folgenden Schritte umfasst:
(a) Suspendieren von amorphem 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat in einem Lösungsmittel oder Lösungsmittelgemisch bei Temperaturen von mindestens 50°C und
(b) langsames Abkühlen der Suspension.

8. Verfahren nach Anspruch 7, in dem das langsame Abkühlen stufenweise vorgenommen wird.

9. Verfahren nach Anspruch 7 oder 8, in dem das verwendete Lösungsmittel oder Lösungsmittelgemisch ausgewählt ist unter Isopropanol, Toluol, Methyl-THF Diethylcarbonat, Chlorbenzol, n-Butylacetat und i-Butylacetat, n-Butanol, Ethanol, Malonsäureethylester, Methyl-t.butylether, Mischungen aus Toluol und Butanol, Toluol und n-Butylacetat, Malonsäureethylester und Methyl-t.-butylether, und Butylacetat und Methyl-t.-butylether.

10. Verfahren nach Anspruch 7 oder 8, in dem das verwendete Lösungsmittel Isopropanol ist.

## Claims

1. Crystalline form of 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulphonate, **characterized in that** its X-ray powder diffractogram at 25°C has at least 3 of the following 2Θ (2-theta) values:
| |
|---|
| 2Θ = 7.75° ± 0.2 |
| 2Θ = 8.89° ± 0.2 |
| 2Θ = 10.91° ± 0.2 |
| 2Θ = 11.45° ± 0.2 |
| 2Θ = 12.72° ± 0.2 |
| 2Θ = 13.46° ± 0.2 |
| 2Θ = 14.54° ± 0.2 |
| 2Θ = 14.75° ± 0.2 |
| 2Θ = 15.53° ± 0.2 |
| 2Θ = 15.90° ± 0.2 |
| 2Θ = 16.33° ± 0.2 |
| 2Θ = 16.65° ± 0.2 |
| 2Θ = 17.49° ± 0.2 |
| 2Θ = 17.71° ± 0.2 |
| 2Θ = 17.89° ± 0.2 |
| 2Θ = 18.27° ± 0.2 |
| 2Θ = 18.47° ± 0.2 |
| 2Θ = 18.86° ± 0.2 |
| 2Θ = 18.99° ± 0.2 |
| 2Θ = 19.18° ± 0.2 |
| 2Θ = 19.63° ± 0.2 |
| 2Θ = 20.03° ± 0.2 |
| 2Θ = 20.22° ± 0.2 |
| 2Θ = 20.53° ± 0.2 |
| 2Θ = 21.11° ± 0.2 |
| 2Θ = 21.32° ± 0.2 |
| 2Θ = 21.50° ± 0.2 |
| 2Θ = 21.84° ± 0.2 |
| 2Θ = 22.01° ± 0.2 |
| 2Θ = 22.28° ± 0.2 |
| 2Θ = 22.61° ± 0.2 |
| 2Θ = 23.05° ± 0.2 |
| 2Θ = 23.39° ± 0.2 |
| 2Θ = 24.02° ± 0.2 |
| 2Θ = 24.22° ± 0.2 |
| 2Θ = 24.62° ± 0.2 |
| 2Θ = 24.97° ± 0.2 |
| 2Θ = 25.59° ± 0.2 |
| 2Θ = 25.99° ± 0.2 |
| 2Θ = 26.31° ± 0.2 |
| 2Θ = 26.67° ± 0.2 |
| 2Θ = 26.85° ± 0.2 |
| 2Θ = 27.07° ± 0.2 |
| 2Θ = 27.43° ± 0.2 |
| 2Θ = 28.21° ± 0.2 |
| 2Θ = 28.34° ± 0.2 |
| 2Θ = 28.78° ± 0.2 |
| 2Θ = 29.59° ± 0.2 |
| 2Θ = 30.19° ± 0.2 |
| 2Θ = 30.37° ± 0.2 |
| 2Θ = 30.96° ± 0.2 |
| 2Θ = 31.29° ± 0.2 |
| 2Θ = 31.70° ± 0.2 |
| 2Θ = 31.81° ± 0.2 |
| 2Θ = 32.16° ± 0.2 |
| 2Θ = 33.02° ± 0.2 |
| 2Θ = 33.39° ± 0.2 |
| 2Θ = 33.64° ± 0.2 |
| 2Θ = 33.79° ± 0.2 |
| 2Θ = 33.80° ± 0.2 |
| 2Θ = 34.20° ± 0.2 |
| 2Θ = 34.77° ± 0.2 |
| 2Θ = 34.89° ± 0.2 |
| 2Θ = 35.66° ± 0.2 |
| 2Θ = 35.95° ± 0.2 |
| 2Θ = 36.67° ± 0.2 |
| 2Θ = 37.03° ± 0.2 |
| 2Θ = 37.38° ± 0.2 |
| 2Θ = 37.51° ± 0.2 |
| 2Θ = 37.87° ± 0.2 |

2. Crystalline form according to Claim 1, **characterized in that** its X-ray powder diffractogram at 25°C has at least 4 of the 2Θ values specified in Claim 1.

3. Composition comprising the crystalline form according to Claim 1 or 2 and suitable auxiliaries.

4. Use of the crystalline form according to Claim 1 or 2 or the composition according to Claim 3 for preparing compositions for combating plant-pathogenic harmful fungi in and/or on plants or in and/or on plant seeds.

5. Use of the crystalline form according to Claim 1 or 2 or the composition according to Claim 3 for the treatment of seeds.

6. Process for combating plant-pathogenic harmful fungi in and/or on plants or in and/or on plant seeds, **characterized in that** the crystalline form according to Claim 1 or 2 or the composition according to Claim 3 is applied to the plant-pathogenic harmful fungi and/or their habitat and/or seeds.

7. Method for preparing the compound as defined in Claim 1 or 2 comprising the following steps:
(a) suspending amorphous 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulphonate in a solvent or solvent mixture at temperatures of at least 50°C and
(b) slow cooling of the suspension.

8. Method according to Claim 7 in which the slow cooling is carried out in stages.

9. Method according to Claim 7 or 8, in which the solvent or solvent mixture used is selected from isopropanol, toluene, methyl-THF, diethyl carbonate, chlorobenzene, n-butyl acetate and isobutyl acetate, n-butanol, ethanol, ethyl malonate, methyl t-butyl ether, mixtures of toluene and butanol, toluene and n-butyl acetate, ethyl malonate and methyl t-butyl ether, and butyl acetate and methyl t-butyl ether.

10. Method according to Claim 7 or 8 in which the solvent used is isopropanol.

## Revendications

1. Forme cristalline de méthane-sulfonate de 2-{3-[2-(1-{[3,5-bis(difluorométhyl)-1H-pyrazol-1-yl]acétyl}pipéridin-4-yl)-1,3-thiazol-4-yl]-4,5-dihydro-1,2-oxazol-5-yl}-3-chlorophényle, **caractérisée en ce que** son diffractogramme de rayons X sur poudre à 25 °C présente au moins 3 des valeurs 2θ (2-thêta) suivantes :
| |
|---|
| 2θ = 7,75° ± 0,2 |
| 2θ = 8,89 ± 0,2 |
| 2θ = 10,91 ± 0,2 |
| 2θ = 11,45 ± 0,2 |
| 2θ = 12,72 ± 0,2 |
| 2θ = 13,46 ± 0,2 |
| 2θ = 14,54 ± 0,2 |
| 2θ = 14,75 ± 0,2 |
| 2θ = 15,53 ± 0,2 |
| 2θ = 15,90 ± 0,2 |
| 2θ = 16,33 ± 0,2 |
| 2θ = 16,65 ± 0,2 |
| 2θ = 17,49 ± 0,2 |
| 2θ = 17,71 ± 0,2 |
| 2θ = 17,89 ± 0,2 |
| 2θ = 18,27 ± 0,2 |
| 2θ = 18,47 ± 0,2 |
| 2θ = 18,86 ± 0,2 |
| 2θ = 18,99 ± 0,2 |
| 2θ = 19,18 ± 0,2 |
| 2θ = 19,63 ± 0,2 |
| 2θ = 20,03 ± 0,2 |
| 2θ = 20,22 ± 0,2 |
| 2θ = 20,53 ± 0,2 |
| 2θ = 21,11 ± 0,2 |
| 2θ = 21,32 ± 0,2 |
| 2θ = 21,50 ± 0,2 |
| 2θ = 21,84 ± 0,2 |
| 2θ = 22,01 ± 0,2 |
| 2θ = 22,28 ± 0,2 |
| 2θ = 22,61 ± 0,2 |
| 2θ = 23,05 ± 0,2 |
| 2θ = 23,39 ± 0,2 |
| 2θ = 24,02 ± 0,2 |
| 2θ = 24,22 ± 0,2 |
| 2θ = 24,62 ± 0,2 |
| 2θ = 24,97 ± 0,2 |
| 2θ = 25,59 ± 0,2 |
| 2θ = 25,99 ± 0,2 |
| 2θ = 26,31 ± 0,2 |
| 2θ = 26,67 ± 0,2 |
| 2θ = 26,85 ± 0,2 |
| 2θ = 27,07 ± 0,2 |
| 2θ = 27,43 ± 0,2 |
| 2θ = 28,21 ± 0,2 |
| 2θ = 28,34 ± 0,2 |
| 2θ = 28,78 ± 0,2 |
| 2θ = 29,59 ± 0,2 |
| 2θ = 30,19 ± 0,2 |
| 2θ = 30,37 ± 0,2 |
| 2θ = 30,96 ± 0,2 |
| 2θ = 31,29 ± 0,2 |
| 2θ = 31,70 ± 0,2 |
| 2θ = 31,81 ± 0,2 |
| 2θ = 32,16 ± 0,2 |
| 2θ = 33,02 ± 0,2 |
| 2θ = 33,39 ± 0,2 |
| 2θ = 33,64 ± 0,2 |
| 2θ = 33,79 ± 0,2 |
| 2θ = 33,80 ± 0,2 |
| 2θ = 34,20 ± 0,2 |
| 2θ = 34,77 ± 0,2 |
| 2θ = 34,89 ± 0,2 |
| 2θ = 35,66 ± 0,2 |
| 2θ = 35,95 ± 0,2 |
| 2θ = 36,67 ± 0,2 |
| 2θ = 37,03 ± 0,2 |
| 2θ = 37,38 ± 0,2 |
| 2θ = 37,51 ± 0,2 |
| 2θ = 37,87 ± 0,2 |

2. Forme cristalline selon la revendication 1, **caractérisée en ce que** son diffractogramme de rayons X sur poudre à 25 °C présente au moins 4 des valeurs 2θ indiquées dans la revendication 1.

3. Composition comprenant la forme cristalline selon la revendication 1 ou 2 et des adjuvants appropriés.

4. Utilisation de la forme cristalline selon la revendication 1 ou 2 ou de la composition selon la revendication 3 pour la fabrication d'agents pour lutter contre des champignons phytopatogènes dans et/ou sur des plantes ou dans et/ou sur des graines de plantes.

5. Utilisation de la forme cristalline selon la revendication 1 ou 2 ou de la composition selon la revendication 3 pour le traitement de graines.

6. Procédé de lutte contre des champignons phytopatogènes dans et/ou sur des plantes ou dans et/ou sur des graines de plantes, **caractérisé en ce que** la forme cristalline selon la revendication 1 ou 2 ou la composition selon la revendication 3 est appliquée sur les champignons phytopatogènes et/ou leur habitat et/ou les graines.

7. Procédé de fabrication du composé tel que défini dans la revendication 1 ou 2, qui comprend les étapes suivantes :
(a) la suspension de méthane-sulfonate de 2-{3-[2-(1-{[3,5-bis(difluorométhyl)-1H-pyrazol-1-yl]acétyl}pipéridin-4-yl)-1,3-thiazol-4-yl]-4,5-dihydro-1,2-oxazol-5-yl}-3-chlorophényle amorphe dans un solvant ou un mélange de solvants à des températures d'au moins 50 °C, et
(b) le refroidissement lent de la suspension.

8. Procédé selon la revendication 7, dans lequel le refroidissement lent est réalisé graduellement.

9. Procédé selon la revendication 7 ou 8, dans lequel le solvant ou mélange de solvants utilisé est choisi parmi l'isopropanol, le toluène, le méthyl-THF, le carbonate de diéthyle, le chlorobenzène, l'acétate de n-butyle et l'acétate d'i-butyle, le n-butanol, l'éthanol, l'ester éthylique de l'acide malonique, l'éther de méthyle et de t.-butyle, les mélanges de toluène et de butanol, de toluène et d'acétate de n-butyle, d'ester éthylique de l'acide malonique et d'éther de méthyle et de t.-butyle, et d'acétate de butyle et d'éther de méthyle et de t.-butyle.

10. Procédé selon la revendication 7 ou 8, dans lequel le solvant utilisé est l'isopropanol.
